(19) Europäisches Patentamt / European Patent Office / Office européen des brevets

(11) **EP 3 094 308 B1**

(12) **EUROPEAN PATENT SPECIFICATION**

(45) Date of publication and mention
of the grant of the patent:
**26.12.2018   Bulletin 2018/52**

(21) Application number: **15702962.0**

(22) Date of filing: **19.01.2015**

(51) Int Cl.:
*A61K 8/64* (2006.01)          *A61Q 11/00* (2006.01)
*A61K 8/73* (2006.01)          *A61K 38/17* (2006.01)
*A61K 6/00* (2006.01)          *A61K 6/027* (2006.01)

(86) International application number:
**PCT/EP2015/000079**

(87) International publication number:
**WO 2015/106970 (23.07.2015 Gazette 2015/29)**

(54) **PROCESS FOR PRODUCING EMD OF INCREASED STABILITY**

PROZESS ZUR HERSTELLUNG ZAHNEMAILLEMATRIXPROTEINE VON ERHÖHTER STABILITÄT

PROCÉDURE DE PRODUIRE LES PROTEINES DE MATRIX D'ÉMAIL DENTAIRE À UNE STABILITÉ
AUGMENTÉE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **17.01.2014   SE 1450046
08.05.2014   SE 1450546**

(43) Date of publication of application:
**23.11.2016   Bulletin 2016/47**

(73) Proprietor: **Straumann Holding AG
4002 Basel (CH)**

(72) Inventors:
• **APICELLA, Alessandra
1004 Lausanne (CH)**
• **HEUNEMANN, Peggy
5415 Obersiggenthal (CH)**

(74) Representative: **Valea AB
Box 7086
103 87 Stockholm (SE)**

(56) References cited:
**WO-A1-00/53197          WO-A2-2006/064381
US-A1- 2002 169 105**

**Description**

**FIELD OF THE INVENTION**

The present invention relates to a process for producing a pharmaceutical, dental and/or cosmetic formulation comprising purified enamel matrix proteins and/or enamel matrix derivative (EMD) proteins, which is heat stable. In particular, the present invention is related to a process for producing stable formulations of enamel matrix proteins and/or enamel matrix derivative (EMD) proteins, wherein said proteins and/or derivatives are solved in a low pH formulation before being subjected to a heat-treatment step.

**BACKGROUND OF THE INVENTION**

Enamel matrix proteins, present in the enamel matrix, are most well-known as precursors to enamel. Prior to cementum formation, enamel matrix proteins are deposited on the root surface at the apical end of the developing tooth-root. There is evidence that the deposited enamel matrix is the initiating factor for the formation of cementum. Again, the formation of cementum in itself is associated with the development of the periodontal ligament and the alveolar bone. Enamel matrix proteins can therefore promote periodontal regeneration through mimicking the natural attachment development in the tooth (Gestrelius S, Lyngstadaas SP, Hammarstrøm L. Emdogain - periodontal regeneration based on biomimicry. Clin Oral Invest 4:120-125 (2000)).

Isolated enamel matrix proteins are able to induce not only one but an orchestrated cascade of factors, naturally found in tissues developing adjacent to the enamel matrix. They mimic the natural environment of a developing tissue and thus mimic a natural stimulation for tissue regeneration, cell differentiation and/or maturation.

Enamel matrix derivative (EMD), in the form of a purified acid extract of proteins from pig enamel matrix, has previously been successfully employed to restore functional periodontal ligament, cementum and alveolar bone in patients with severe tooth attachment loss (Hammarström et al., 1997, Journal of Clinical Periodontology 24, 658-668).

Furthermore, in studies on cultured periodontal ligament cells (PDL), it was shown that the attachment rate, growth and metabolism of these cells were significantly increased when EMD was present in the cultures. Also, cells exposed to EMD showed increased intracellular cAMP signalling and autocrine production of growth factors, when compared to controls. Epithelial cells on the other hand, although increasing cAMP signalling and growth factor secretion when EMD was present, were inhibited in both proliferation and growth (Lyngstadaas et al., 2001, Journal of Clinical Periodontology 28, 181-188).

Enamel matrix proteins and enamel matrix derivatives (EMD) have previously been described in the patent literature to be able to induce hard tissue formation (i.e. enamel formation, US Patent No. 4,672,032 (Slavkin)), endorse binding between hard tissues (EP-B-0 337 967 and EP-B-0 263 086), promote open wound healing, such as of skin and mucosa, have a beneficial effect on treatment of infections and inflammatory diseases (EP-1059934 and EP-01201915.4), repair mineralized and non-mineralized tissue, such as bone, cartilage, teeth, soft tissue and mucosa, treat a condition involving inflammation or infection (WO 06/64381), induce regeneration of dentin (WO 01/97834), promote the take of a graft (WO 00/53197), induce apoptosis in the treatment of neoplasms (WO 00/53196), regulate imbalance in an immune response to a systemic infection or inflammation (WO 03/024479), and to facilitate filling a wound cavity and/or tissue defect following from a procedure and/or trauma, such as a cytoreductive surgery (WO 02/080994).

EMD is composed of a number of proteins, such as amelogenins, enamelin, tuft protein, proteases, and albumin. Amelogenins, a major constituent of EMD, at least up to 60 % - 90 %, such as 70 - 90 %, are a family of hydrophobic proteins derivable from a single gene by alternative splicing and controlled post secretory processing. They are highly conserved throughout vertebrate evolution and demonstrate a high overall level of sequence homology among all higher vertebrates examined (80%). In fact, the sequences of porcine and human amelogenin gene transcript differ only in 4% of the bases. Thus, enamel matrix proteins and/or EMD proteins, although of porcine origin, are considered "self" when encountered in the human body and can promote dental regeneration in humans without triggering allergic responses or other undesirable reactions. Enamel Matrix Derivative Protein (EMD) is the most known precursor to enamel. Its aqueous solution thickened with PGA is commercialized under the trade-name Straumann® Emdogain®.

EMD is the active component of Straumann Emdogain® gel, used in regenerative therapy for patients suffering from periodontal disease. In this formulation, propylene glycol alginate (PGA) acts as a carrier for the release of EMD onto the affected tissue. Once Emdogain® has been applied, i.e. under conditions of roughly neutral pH, the EMD, which is close to its isoelectric point (pH 6.8), precipitates and forms a compact layer that promotes cell proliferation and ligament extension, resulting in relatively rapid tissue regrowth. Individuals treated with Emdogain® have experienced significant bone fill, and regain of clinical attachment and alveolar bone has been shown to continue for more than a year after treatment. However, it is important that EMD remains stable or shows a predictable evolution in properties during storage, if precipitation prior to application is to be avoided.

In order to keep the EMD in aqueous solution, the solution should have a pH well-below the protein isoelectric point (IEP). For EMD, the IEP is pH 6.5, hence, more preferred is that the pH of the solution is about 5.0. In general, the durability of the solution is determined by the pH value at which the EMD is still capable to precipitate onto the tooth surface. It is considered that the precipitation occurs in physiological conditions, i.e. pH near IEP. Then again, for easy application, the solution is thickened by PGA. The pH and buffering capacity of both the tooth root environment and the EMD-PGA mixture influences the precipitation behavior of the EMD proteins.

Chemically, the PGA is an ester of alginic acid, which is derived from kelp (seaweed). Some of the carboxyl groups are esterified with propylene glycol, some are neutralized with an appropriate alkali, and some remain free. The PGA itself is acidic and the pH of the EMD solution thus decreases after the PGA is dissolved in the EMD solution. What is more, under acidic conditions, the pH keeps decreasing over time, due to the natural degradation of the PGA. Thus, due to the intrinsic properties of the added PGA, it is necessary to start out with a relatively high pH in the solution to enable a longer shelf-life of the product.

Propylene glycol alginates shows about 40% loss in viscosity after a year at 25°C and also becomes less soluble. Ammonium alginate is generally less stable than any of the above. Alginic acid is the least stable of the products and any long chain material degrades to shorter chains within a few months at ambient temperatures. However, alginates comprising short chain material are stable and alginic acid with a degree of polymerization (DP) of about 40 units of uronic acid per chain will show very little change over a year at a temperature of 20°C. However, the main use of alginic acid, as a disintegrant in pharmaceutical tablets, depends on its ability to swell when wetted and this is not affected by changes in DP. The commercial alginates should therefore be stored at a neutral pH in a cool place, i.e. at temperatures of 25°C or lower, as elevated temperatures and an acidic pH can cause significant depolymerization, affecting the commercially useful properties, such as viscosity and gel strength. Said alginates usually contain 10-13% moisture and the rate of depolymerization increases as the proportion of moisture is increased, thus the storage area should be dry.

As mentioned above, EMD proteins have prior been formulated in an aqueous solution with PGA, wherein the degradation of PGA provides acidic products, which in turn decreases the pH over time. At elevated temperatures, the degradation accelerates. In order to avoid the accelerated acidification of the product, and thus to limit this effect, the products have to be transported and stored at low temperatures, i.e. at a temperature range of 2 to 8°C.

What is more, in the commercially available Straumann Emdogain® formulation, enamel matrix protein (EMD) is after its isolation heated for 3 hours at about 80°C in order to inactivate residual proteases. This necessary heat-treatment has recently been found to be a key factor in bringing on an irreversible degradation and denaturation of EMD itself, in turn contributing to a shorter shelf-life and/or less activity of the formulation.

Given its relatively high amelogenin content, EMD self-assembly is expected to involve hierarchical structures based on nanoscale spherical agglomerates, referred to in what follows as "nanospheres". However, such structures are generally only marginally stable, and are hence sensitive to environmental stresses, such as changes in temperature or pH. These may result in irreversible unfolding, leading in turn to an increase of the solubility in proteins generally being most stable in their native folded state. In either case, the proteins will no longer be able to carry out their functions and may eventually precipitate early. What is more, sometimes, aggregation may also happen between partially unfolded species, leading to a limited efficacy of the product. Hydrophobic contacts are known to be largely responsible for intermolecular interactions and aggregation in proteins, but charged residues may also play an important role. Interaction of these latter with positively charged amino acids may therefore provide a means of limiting unfolding and/or early precipitation. Clearly any such aggregation suppressor should not interfere with the protein conformations and regenerative properties of the EMD. Moreover, the choice of additive should be compatible with the clinical requirements of dental applications.

## DISCLOSURE OF THE INVENTION

As is clearly demonstrated in the experimental section, the stability of EMD in an aqueous environment was investigated using pH variations and thermal stress to accelerate protein aggregation and unfolding (Casal HL, Kohler U, Mantsch HH. Structural and conformational changes of β-lactoglobulin B : an infrared spectroscopic study of the effect of pH and temperature. Biochimica et Biohysica Acta (BBA)- Protein structure and Molecular Enzymology 1998 ; 957(1) : 11-20.).The results were then further compared with results obtained in the presence of arginine in order to evaluate either pH and/or arginine effectiveness in limiting EMD intermolecular interactions and hence the extent of denaturation, aggregation, precipitation, unfolding and/or refolding. Finally, the influence of arginine on the precipitation kinetics of EMD and EMD/PGA was assessed using the stopped flow (SF) turbidity method. The findings provide new and surprising insight into the EMD unfolding/refolding process and evidence that the use of low pH, and/or arginine, as a stabilizing additive, for EMD-based products has the potential to extend significantly their effective shelf-life.

The present invention thus for the first time describes a process for producing an EMD formulation with improved stability and hence prolonged regenerative capacity of the EMD formulation during storage.

The present invention for the first time describes a process for producing a pharmaceutical, dental and/or cosmetic formulation comprising enamel matrix proteins and/or enamel matrix derivative (EMD) proteins and a suitable pharmaceutical carrier, which is characterized by a shelf life and/or durability of at least 12 months at 2°C-RT, such as of at least 12 months at RT.

The process for producing said formulation comprising enamel matrix proteins and/or enamel matrix derivative (EMD) proteins and a suitable pharmaceutical carrier typically comprises the following steps:

a. isolating enamel matrix proteins and/or enamel matrix derivative (EMD) proteins from a developing mammals teeth,
b. solving said isolate in a suitable pharmaceutical carrier,
c. lowering the pH of said formulation initially to below or at the most pH 4, and
d. heating said formulation to between 20-100°C for at least 10 minutes.

In one embodiment, the pH of the formulation is in step c. lowered to below pH 4, 3.9, 3.8, 3.7, 3.6, or below 3.5, or to at most 4, such as to 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9 or 4.

The pH of the formulation can in step c. alternatively be lowered to between pH4-pH2, such as to between pH4- pH3.5, or such as to between pH3.5-pH 2.

The formulation of step c. is in step d. heated to 20-100°C, such as to 20, 30, 40, 50, 60, 70, 80, 90 or 100°C for at least 10 minutes. In one embodiment, formulation of step c. is in step d. heated to at least 80°C for at least 1-3 hours, such as to at least 80°C for at least 3 hours, or to at least 50°C for at least 1 hour, or to at least 20°C for at least 1 hour.

Step d. can be repeated one or several times, if necessary, to achieve the desired result of inactivating inherent proteases and/or residual contaminants in the formulation.

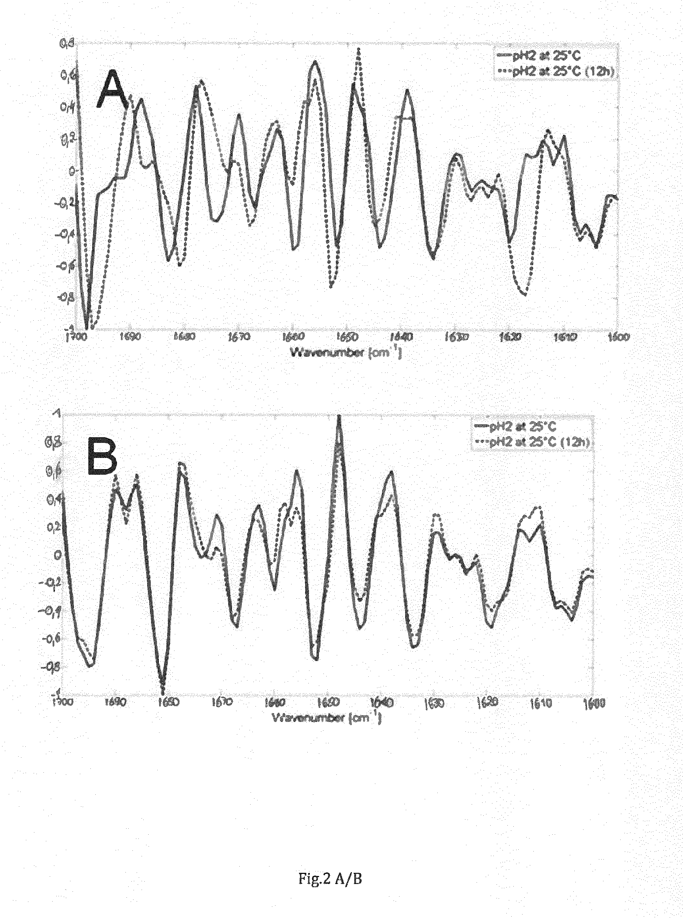

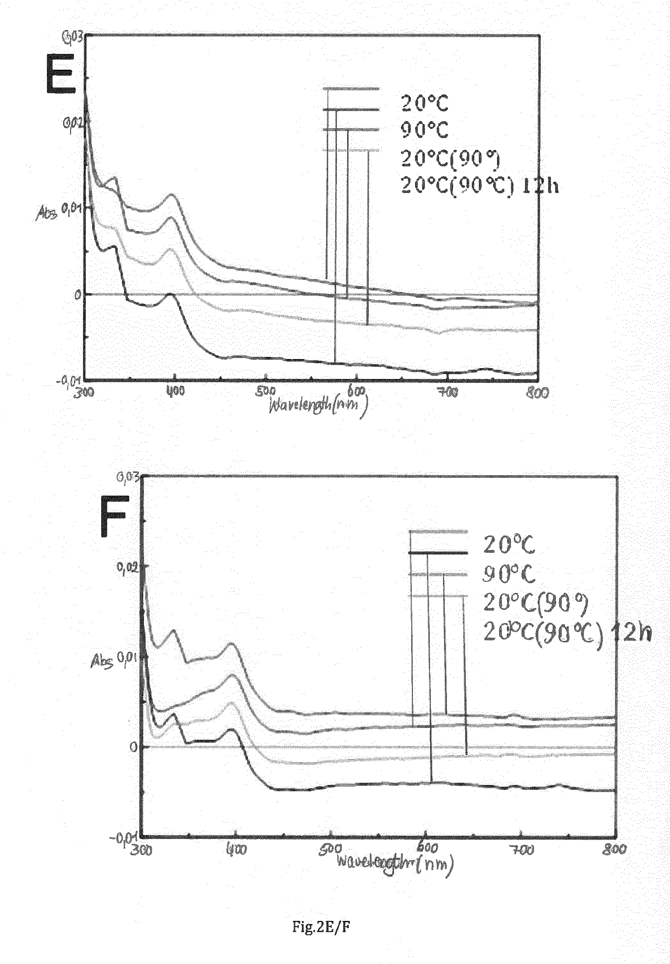

As is clearly demonstrated in the experimental section, (see Fig 2A), after heat treatment and incubation for 12 h at 25 °C, EMD showed significant conformational changes of the α-helices, while the native β-sheets were not greatly affected by the heat treatment. I.e., substantial aggregation of EMD could be observed.

Addition of arginine at pH 2 led to findings indicating the conformations associated with the α-helices to remain stable even after heat treatment. Moreover, β-sheets remained unchanged after heat treatment, which suggests limited conformational change of the exposed surface to have taken place. The arginine is therefore inferred to bind to external aggregation sites of the EMD proteins, which may include β-turns. Moreover, in neutralizing the EMD binding sites, arginine is expected to promote compactness of the protein structure, also seen in an increase of nanosphere size. Taken together, these results imply arginine to stabilize the EMD structure at pH 2 with respect to heat treatment.

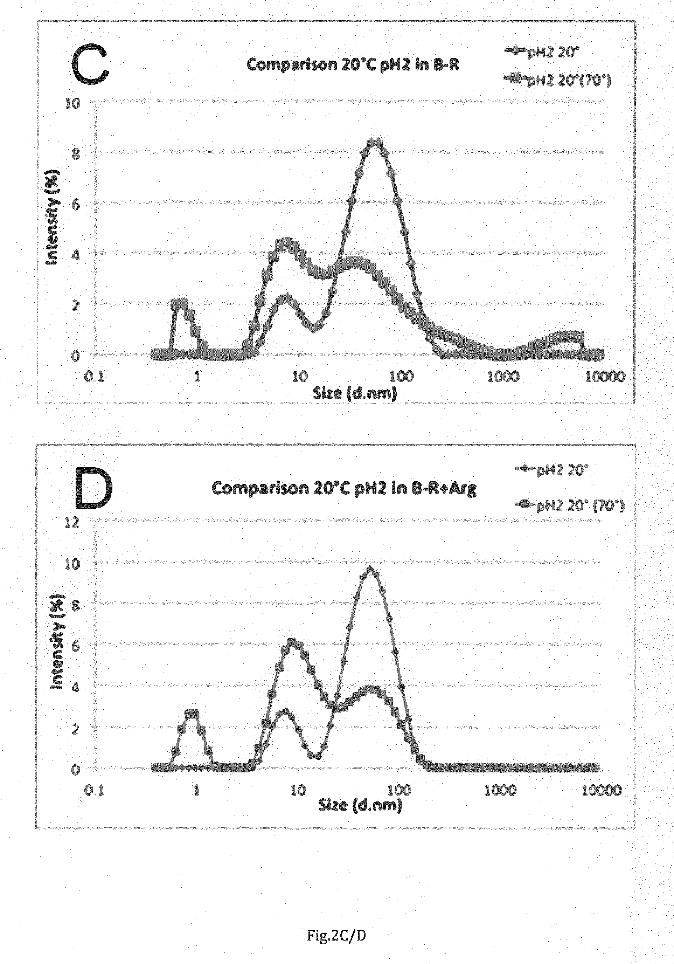

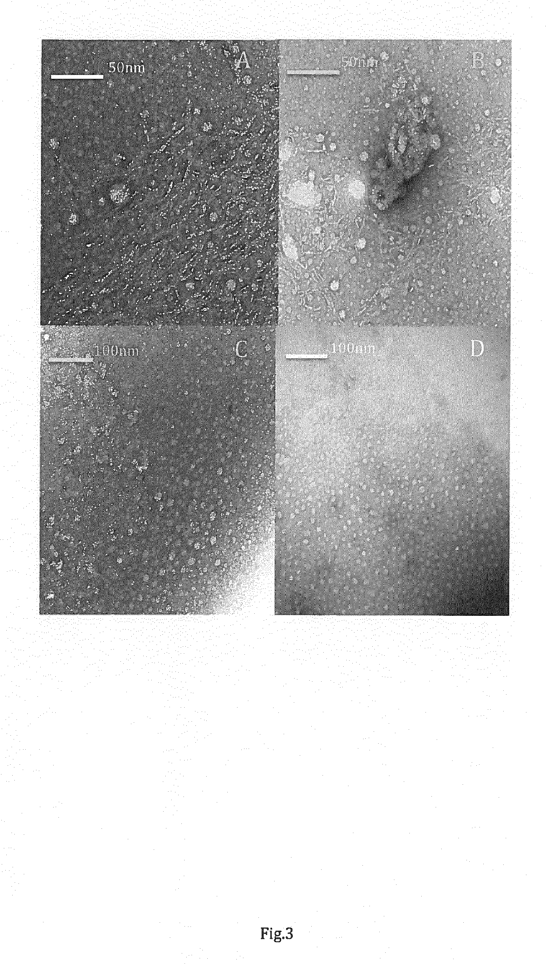

The EMD particle size distribution at pH 2 in the absence of arginine (see Fig 2C) demonstrated that heat treatment up to 70 °C and cooling to 20 °C lead to conformational changes (see also the TEM micrographs in Fig 3A-B). Nanofilaments and nanospheres of about 70 nm or less in diameter, typical of amelogenin in its native state (7), are visible in Fig 3A, but, as seen in Fig 3B, these were partially aggregated after heat treatment. The aggregation after heat treatment was absent in the presence of arginine at pH 2. Given that denaturation of the EMD did not occur in presence of arginine, it is concluded that heat-induced unfolding was absent at pH 2. Hence, the nanosphere structure of the EMD remained substantially intact (Fig 3C-D).

Nonetheless, EMD solved in low pH, such as in pH 2, measured at 20 and 90 °C, and at 20 °C after heat treatment suggests that temperature-induced aggregation was largely reversed on cooling (exepriments not shown)

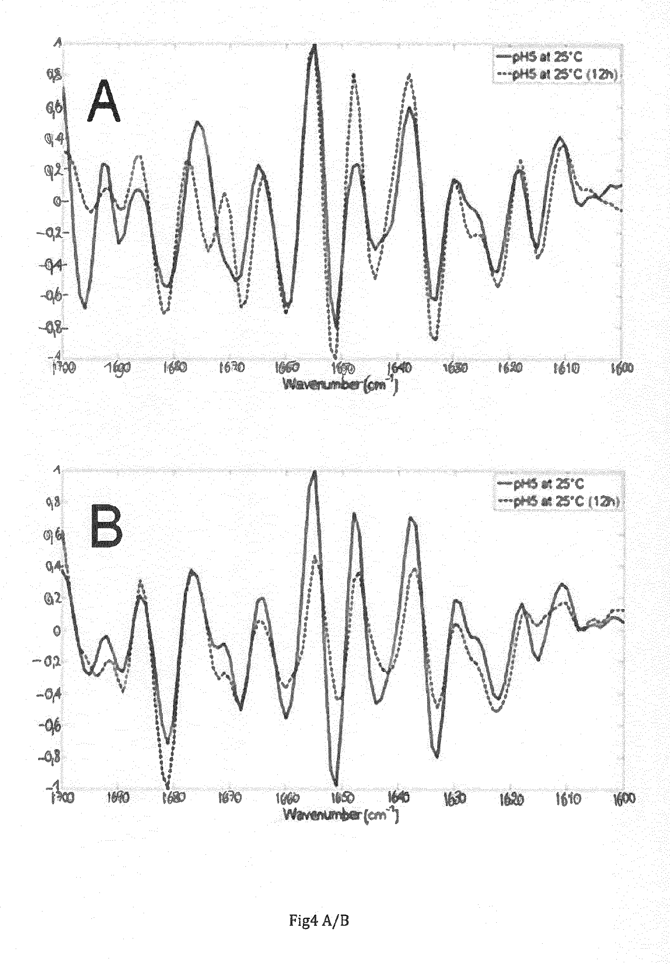

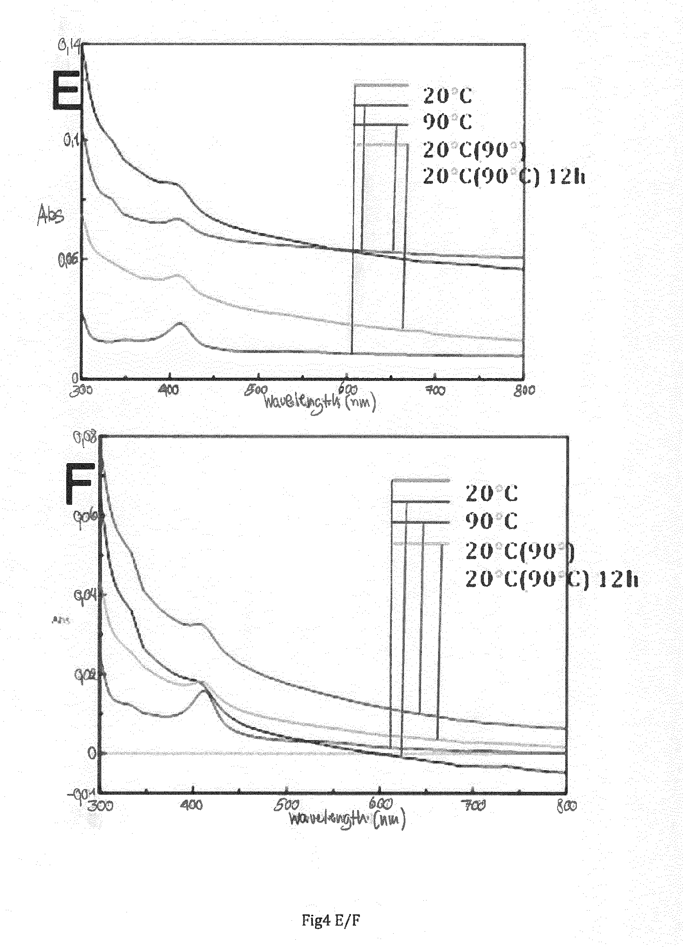

As is shown in Fig 4A, heat treatment of EMD formulations with pH 5 resulted in a significantly modified molecular organization of the EMD.

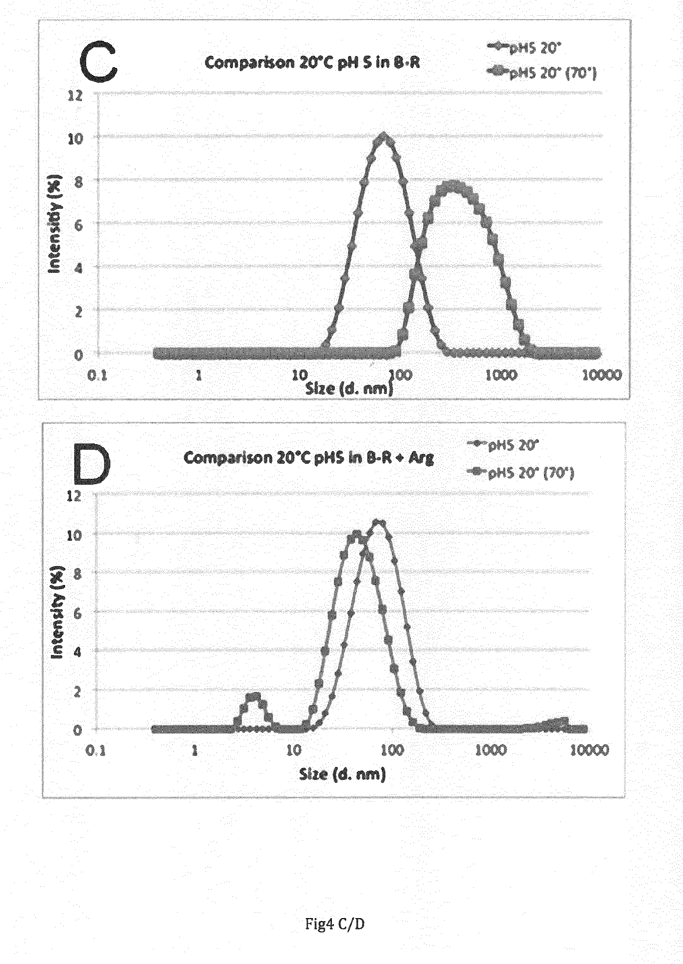

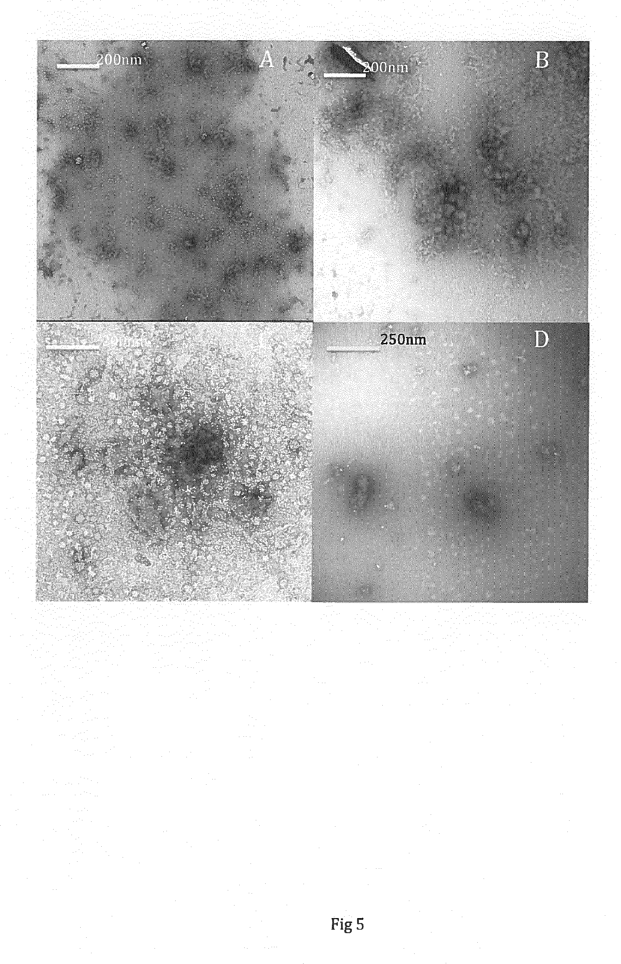

As can be seen in Fig 4C, heat treatment of formulations with EMD at pH 5 led to increased aggregation (see also Fig 5A-B). Nanofilaments and isolated nanospheres were initially present (Fig 5A), but extensive agglomeration was observed after heat treatment (Fig 5B). The nanospheres, whose dimensions in the as-prepared EMD were again consistent with those typical of amelogenin, were assumed to aggregate during heat treatment. As seen from Fig 4D, arginine limits this aggregation.

Hence, as observed at pH 2, heat treatment of formulations of EMD at pH 5 may result in partial disaggregation of the nanospheres. This was reflected in Fig 5C-D, which suggests nanofilaments initially present in the specimens to have broken up into nanospheres and even smaller fragments after heat treatment.

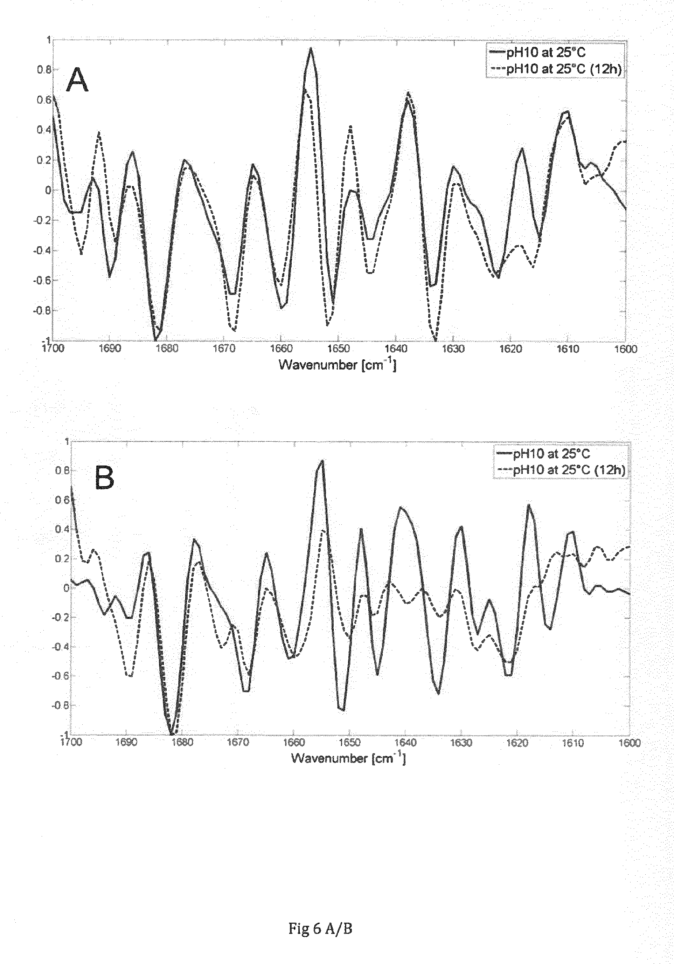

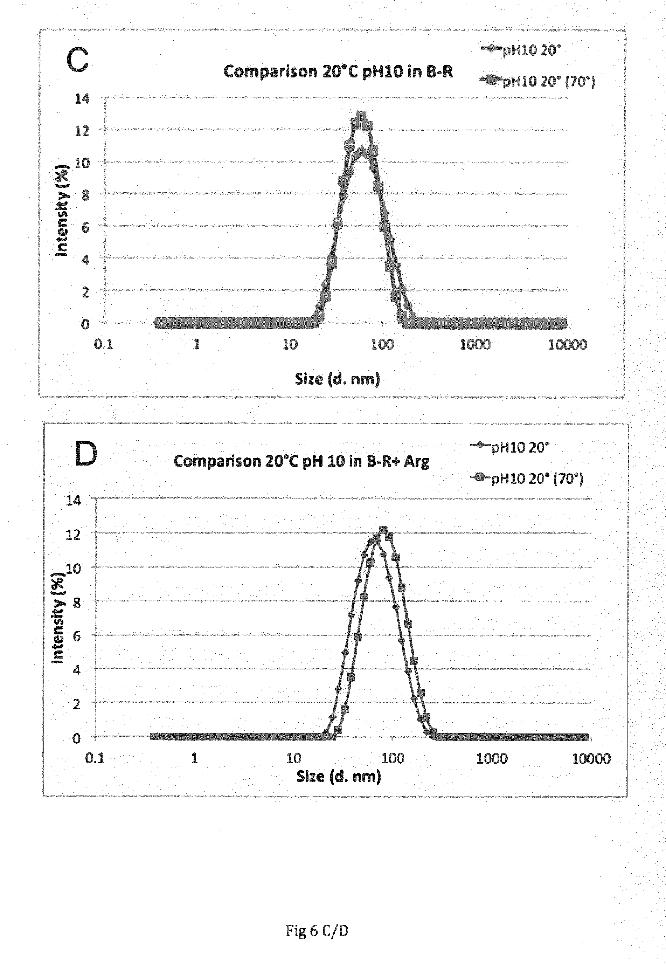

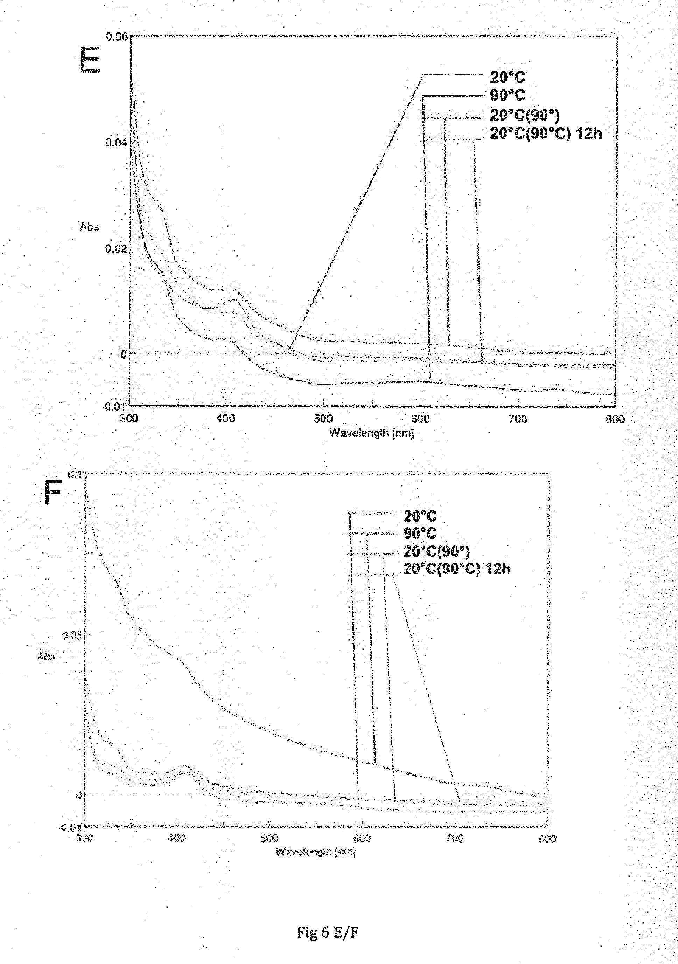

In contrast, at pH 10, the protein conformations are strongly modified with respect to the native conformations seen at lower pH (Fig 6A). Moreover, the combination of high pH and ionic charges stabilizes the secondary structure during heat treatment in the absence of arginine. In the presence of arginine, on the other hand, the spectra obtained before and after heat treatment no longer overlaps; the proportion of random coils is reduced, and peaks corresponding to the β-turn structures are again distinguishable (Fig 6B).

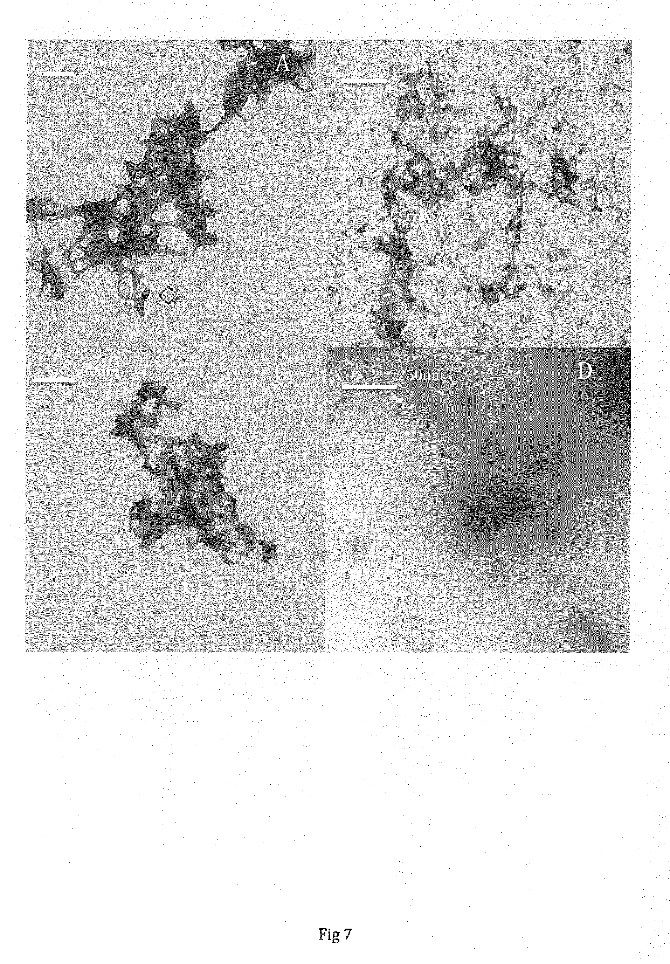

Fig 7 confirms the morphology of the EMD at high pH to be very different to that observed at lower pH, being characterized by extensive amorphous gel-like agglomerates.

The suitable pharmaceutical carrier is typically a universal buffer, such as a Britton-Robinson buffer, a Carmody buffer, an acetic buffer (acetic acid/sodium acetate), a saline buffer, a citric buffer (citric acid/sodium citrate and/or HCl/sodium citrate), or a physical sodium buffer.

The suitable pharmaceutical carrier can also be selected from the group consisting of aqueous acetic acid, citric acid, or $H_2O$.

In one embodiment, acetic acid is used at pH 2- pH 8, such as at pH 5.

In particular, in experimental testing, a Britton-Robinson buffer is used.

The present invention relates to a process for producing a pharmaceutical, dental and/or cosmetic formulation comprising enamel matrix proteins and/or enamel matrix derivative (EMD) proteins and a suitable pharmaceutical carrier, wherein the enamel matrix proteins and/or enamel matrix derivative (EMD) proteins comprise at least 60-100% amelogenin, such as at least 60-70, 60-80, 70-90, 60, 65, 70, 75, 80, 85, 90, 95 or 97%, such as at least 99% amelogenin, having an average molecular weight selected from the group consisting of between 18-25 kDa, such as between 20-24 kDa, between 20- 22 kDa, and 20 kDa.

The present invention further relates to a process for producing a pharmaceutical, dental and/or cosmetic formulation comprising enamel matrix proteins and/or enamel matrix derivative (EMD) proteins and a suitable pharmaceutical carrier, which further comprises mixing the formulation of step d. with a suitable viscosity modifier.

In one embodiment, said process further comprises adjusting the pH of the formulation of step d. to at least pH5 before mixing said formulation with a suitable viscosity modifier, such as PGA. Said PGA can be e-beam sterilized PGA. Preferably, said PGA is sterilized propylene glycol alginate (PGA) with a weight average molecular weight above 130 kDa.

In an alternative embodiment, said process further comprises adjusting the pH of the formulation of step d. to at the most pH5 before mixing said formulation with a suitable viscosity modifier. The pH of said formulation is typically adjusted to between pH 2- pH 5, such as between pH 2.5-pH 4.5, or to between pH2-pH4, such as to pH 2.0, 2.1, 2.2, 2.3, 2.4, 2.5, 2.6, 2.7, 2.8, 2.9, 3.0, 3.3, 3.5, 3.8, 4.0, 4.3, or 4.5.

In said alternative embodiment, the viscosity modifier is selected from the group consisting of hyaluronic acid. The Hyaluronic acid can be e-beam sterilized.

In yet another alternative embodiment, the process of the present invention comprises adjusting the pH of the formulation of step d. to at the most pH 5 without mixing said formulation with any suitable viscosity modifier. The pH of said formulation is typically adjusted to between pH 2- pH 5, such as between pH 2.5-pH 4.5, or to between pH 2-pH 4, such as to pH 2.0, 2.1, 2.2, 2.3, 2.4, 2.5, 2.6, 2.7, 2.8, 2.9, 3.0, 3.3, 3.5, 3.8, 4.0, 4.3, or 4.5.

The present invention also relates to a pharmaceutical, dental and/or cosmetic formulation comprising enamel matrix proteins and/or enamel matrix derivative (EMD) proteins and a suitable pharmaceutical carrier produced by a process according to the present invention.

The present invention thus relates to a pharmaceutical, dental and/or cosmetic formulation comprising enamel matrix proteins and/or enamel matrix derivative (EMD) proteins and a suitable pharmaceutical carrier, which is characterized by a shelf life and/or durability of at least 12 months at 2°C-RT, such as of at least 12 months at RT, and which is produced by a process comprising the following steps:

    a. isolating enamel matrix proteins and/or enamel matrix derivative (EMD) proteins from a developing mammals teeth,
    b. solving said isolate in a suitable pharmaceutical carrier,
    c. lowering the pH of said formulation initially to below or at the most pH 4, and
    d. heating said formulation to between 20-100°C for at least 10 minutes.

A pharmaceutical, dental and/or cosmetic formulation comprising enamel matrix proteins and/or enamel matrix derivative (EMD) proteins and a suitable pharmaceutical carrier produced by a process of the present invention typically comprises enamel matrix proteins and/or enamel matrix derivative (EMD) proteins at a concentration of between 5-35 mg/ml of the formulation, such as at least 5, 10, 15, 20, 25, 29, 30, 31, 32 or 35 mg/ml of the formulation.

In another embodiment, a pharmaceutical, dental and/or cosmetic formulation comprising enamel matrix proteins and/or enamel matrix derivative (EMD) proteins and a suitable pharmaceutical carrier produced by a process of the present invention comprises enamel matrix proteins and/or enamel matrix derivative (EMD) proteins at a concentration of between 100μ-5 mg/ml of the formulation, such as at least 100, 150, 250, 500 μg/ml of the formulation, or such as at least 1, 2, 3, 4, or 5mg/ml of the formulation.

A pharmaceutical, dental and/or cosmetic formulation comprising enamel matrix proteins and/or enamel matrix derivative (EMD) proteins and a suitable pharmaceutical carrier produced by a process of the present invention can further comprise arginine. In one embodiment, said arginine has a concentration of 1-500 mM when it is added to the formulation, such as at the least 1, 10, 100, 250, 300, 400 or 500 mM. In another embodiment, said arginine has a concentration of 1, 10, 100, 250, 300, 400, 450, or 500 mM. Typically, the concentration of arginine in the formulation is at the most 500mM.

Arginine is common naturally occurring amino acid. Its structure, its ability to form H-bonds and its charge distribution under physiological conditions are such that arginine readily binds to negatively charged groups and is also able to interact strongly with polar environments, such as water. Arginine has indeed proven to be effective as an additive for the prevention of protein aggregation, and is also known to have a relatively minor effect on the stability of the native structure in many cases, so that it may be considered to be a non-denaturing reagent.

The present inventors investigated the stability of EMD in an aqueous environment using pH variations and thermal stress to accelerate protein aggregation and unfolding. The results were then compared with results obtained in the presence of low pH and/or arginine in order to evaluate their effectiveness in limiting EMD intermolecular interactions and hence the extent of denaturation, aggregation, precipitation, unfolding and/or refolding. Finally, the influence of low pH and arginine on the precipitation kinetics of EMD and EMD/PGA was assessed using the stopped flow (SF) turbidity method.

The findings leading to the present invention provide new and surprising insight into the EMD unfolding/refolding process and evidence that the use of low pH and/or arginine as a stabilizing additive for EMD-based products has the potential to extend significantly their effective shelf-life.

Using a variety of analytical techniques, the present invention for the first time demonstrates that addition of arginine to aqueous EMD can limit denaturation of EMD at acidic pH, furthermore, it also evidences that addition of arginine to aqueous EMD may reverse large-scale aggregation at high pH. Investigation of the release kinetics of EMD subsequent to a step-like increase in pH to pH 7, which is close to the isoelectric point of the EMD, also showed arginine not to have an adverse influence on the precipitation rates from either aqueous solution or from a PGA gel carrier. Indeed, the response in systems with an initial pH of 5 was similar with arginine to that at pH 2, both with and without arginine, again reflecting the stabilizing effect of arginine on the EMD. The results shown in experiment 1 therefore indicate that arginine has the potential to improve significantly the storage life of EMD/PGA-based gels used in regenerative therapy, without compromising the release of the EMD at the site of application.

The present invention further relates to a pharmaceutical, dental and/or cosmetic formulation comprising enamel matrix proteins and/or enamel matrix derivative (EMD) proteins and a suitable pharmaceutical carrier produced by a process of the present invention which in addition comprises a suitable bone ceramic and/or a bonegraft.

The present invention relates to a pharmaceutical, dental and/or cosmetic formulation comprising enamel matrix proteins and/or enamel matrix derivative (EMD) proteins and a suitable pharmaceutical carrier produced by a process of the present invention for use in medicine.

In particular, a pharmaceutical, dental and/or cosmetic formulation comprising enamel matrix proteins and/or enamel matrix derivative (EMD) proteins and a suitable pharmaceutical carrier produced by a process of the present invention is intended for use in manufacturing a medicament for use in wound-treatment and/or in treatment of periimplant diseases and/or periodontitis or for inducing formation of mineralized tissue.

Also envisioned in the present invention is a method for wound-healing and/or for treatment of periimplant diseases and/or periodontitis or for inducing formation of mineralized tissue, wherein a patient in need thereof is treated with or administered a pharmaceutical, dental and/or cosmetic formulation comprising enamel matrix proteins and/or enamel matrix derivative (EMD) proteins and a suitable pharmaceutical carrier which is produced by a process of the present invention.

A kit is described comprising a pharmaceutical, dental and/or cosmetic formulation according to any one of claims 18-28, and at least one further component selected from the group consisting of granules, bone ceramics, scaffolds, a bonegraft, natural bone material, a bone block, artificial teeth, and an implant.

## DEFINITIONS

"Soft tissues", (i.e. non-mineralised tissues), can in the present context be used interchangeably with gingival tissue, and may be defined as collagen or epithelium containing tissues, including skin and mucosa, muscle, blood and lymph vessels, nerve tissues, glands, tendons, eyes and cartilage. In general, the formulation of the present invention can be used to promote healing of a wound not only in skin and mucosa, but in any gingival tissue of a patient in need thereof.

The term "hard-tissue formation" in "mineralised tissue" may be summarised as the production by cells of an organic matrix capable of accepting mineral, with the activity of the enzyme alkaline phosphatase and a good blood supply prerequisites. In general, the formulation of the present invention can be used to promote hard-tissue formation in a patient in need thereof.

### Active enamel substances

As used herein, "enamel matrix" means a precursor to enamel and may be obtained from any relevant natural source, i.e. a mammal in which teeth are under development. A suitable source is developing teeth from slaughtered animals such as, e.g., calves, pigs or lambs. Another source is e.g. fish skin. In the present context, the term "an active enamel substance" is used to encompass enamel matrix proteins and/or enamel matrix derivative (EMD) proteins non-discriminant of their source.

Enamel matrix can be prepared from developing teeth as described previously (EP-B-0 337 967 and EP-B-0 263 086). The enamel matrix is scraped off and enamel matrix proteins and/or enamel matrix derivative (EMD) proteins are prepared, e.g. by extraction with aqueous solution such as a buffer, a dilute acid or base or a water/solvent mixture, followed by size exclusion, desalting or other purification steps, alternatively followed by freeze-drying. Enzymes may alternatively be deactivated by treatment with heat or solvents, in which case the derivatives may be stored in liquid form without freeze-drying.

As an alternative source of the enamel matrix proteins and/or enamel matrix derivative (EMD) proteins one may also use generally applicable synthetic routes, well known to a person skilled in the art, or use cultivated eukaryotic and/or prokaryotic cells modified by DNA-techniques. The enamel matrix proteins and/or enamel matrix derivative (EMD) proteins may thus be of recombinant origin and alternatively genetically and/or chemically modified (see, e.g., Sambrook, J. et al.: Molecular Cloning, Cold Spring Harbor Laboratory Press, 1989).

In the present context, enamel matrix proteins and/or enamel matrix derivative (EMD) proteins include one or several enamel matrix proteins or parts or fragments of such proteins, produced naturally by alternate splicing or processing, or by either enzymatic or chemical cleavage of a natural length protein, or by synthesis of polypeptides *in vitro* or *in vivo* (e.g. recombinant DNA methods and/or cultivation of diploid cells). Enamel matrix proteins and/or enamel matrix derivative (EMD) proteins also include enamel matrix related polypeptides or proteins. The polypeptides or proteins may be bound to a suitable biodegradable carrier molecule, such as polyamine acids or polysaccharides, or combinations thereof. Furthermore, the term enamel matrix proteins and/or enamel matrix derivative (EMD) proteins also encompass synthetic analogous substances.

Proteins are biological macromolecules constituted by amino acid residues linked together by peptide bonds. Proteins, as linear polymers of amino acids, are also called polypeptides. Typically, proteins have 50-800 amino acid residues and hence have molecular weights in the range of from about 6,000 to about several hundred thousand Dalton or more. Small proteins are called peptides, oligopeptides or polypeptides. In the context of the present invention, a "polypeptide fragment" for use in accordance with the present invention, refers to a polypeptide which may be, but is not limited to, being 1-50 amino acids in length, such as 5, 10, 15, 20, 25, 30, 35, 40, 41, 42, 43, 44, 45, 46, 47, 47, 48, 49 or 50 amino acids. Such polypeptides may also be longer than 50 amino acids.

Enamel matrix proteins are proteins that normally are present in enamel matrix, i.e. the precursor for enamel (Ten Cate: Oral Histology, 1994; Robinson: Eur. J. Oral Science, Jan. 1998, 106 Suppl. 1:282-91), or proteins which can be obtained by cleavage of such proteins. In general, such proteins have a molecular weight below 120,000 Dalton and include amelogenins, non-amelogenins, proline-rich non-amelogenins and tuftelins.

Examples of proteins for use according to the invention are amelogenins, proline-rich non-amelogenins, tuftelin, tuft proteins, serum proteins, salivary proteins, ameloblastin, sheathlin, and derivatives thereof, and mixtures thereof. Moreover, other proteins for use according to the invention are found in the marketed product EMDOGAIN® (BIORA AB, Sweden).

EMDOGAIN® (BIORA AB, S-205 12 Malmö, Sweden) contains 30 mg enamel matrix proteins and/or enamel matrix derivative (EMD) proteins, heated for 3 hours at about 80 °C in order to inactivate residual proteases, and 1 ml Vehicle Solution (Propylene Glycol Alginate), which are mixed prior to application, unless the protein and the vehicle are tested separately. The weight ratio is about 80/8/12 between the main protein peaks at 20, 14 and 5 kDa, respectively.

In general, the major proteins of an enamel matrix are known as amelogenins. They are markedly hydrophobic substances that under physiologically conditions form aggregates. They may carry or be carriers for other proteins or peptides.

None withstanding, in light of the well-known fact that amelogenin is an evolutionary very conservative protein, and the homology in between the species is documented to be high, it is presently envisioned that analogous sequences might be found in rat, human, or mouse enamel matrix proteins, e.g. in amelogenin, which exert similar biological effects, e.g. possess osteogenic activity. The present invention consequently also encompasses analogue sequences to porcine amelogenin, which are at least 80-95% identical to human amelogenin, such as at least 80%, 81%, 82%, 83%, 84%, 85%, 86%, 87%, 88%, 89%, 90%, 91%, 92%, 93%, 94%, 95%, 96%, 97%, 98%, 99%, or 99,5% identical, and which show analogue biological activity.

In the present invention, enamel matrix proteins and/or enamel matrix derivative (EMD) proteins, can be isolated from mammalian tissue, be purified recombinant polypeptide fragments, or polypeptide fragments which are synthetically manufactured. As is well known in the art, a recombinantly produced polypeptide will differ slightly from the endogenous template protein, especially when it is produced in a prokaryotic system. The present invention encloses recombinantly produced polypeptide fragments, which are at least 95% identical to the endogenous human amelogenin, such as at least 80%, 81%, 82%, 83%, 84%, 85%, 86%, 87%, 88%, 89%, 90%, 91%, 92%, 93%, 94%, 95%, 96%, 97%, 98%, 99%, or 99,5% identical, and which show analogue biological activity.

A synthetically manufactured polypeptide on the other hand, as is well known in the art, can of course be designed to carry a diversity of chemical permutations that will not hinder and/or effect its original biological activity, e.g. its anti-inflammatory, or its pro-angiogenetic activity. Consequently, the present invention encloses also synthetically permutated polypeptide fragments which are at least 80-95% identical to human amelogenin, a homologue, analogue, or a pharmaceutically acceptable salt thereof, such as at least 80%, 81%, 82%, 83%, 84%, 85%, 86%, 87%, 88%, 89%, 90%, 91%, 92%, 93%, 94%, 95%, 96%, 97%, 98%, 99%, or 99,5% identical, and which show analogue biological activity.

Additionally, any conservative variant of the sequence of a polypeptide fragment which is at least 80-95% identical to human amelogenin, such as at least 80%, 81%, 82%, 83%, 84%, 85%, 86%, 87%, 88%, 89%, 90%, 91%, 92%, 93%, 94%, 95%, 96%, 97%, 98%, 99%, or 99,5% identical, and which shows analogue biological activity, is by virtue of its functional relationship to said sequences considered to be inside the scope of the present invention.

A conservative variant of a sequence is in the present context defined as an amino acid sequence which is conserved at least such as at least 80%, 81%, 82%, 83%, 84%, 85%, 86%, 87%, 88%, 89%, 90%, 91%, 92%, 93%, 94%, 95%, 96%, 97%, 98%, 99%, or 99,5%, when comparing variants of the same amino acid sequence between different species. The degree of conservation of a variant can, as is well known in the field, be calculated according to its derivation of PAM (see Dayhoff, Schwartz, and Orcutt (1978) Atlas Protein Seq. Struc. 5:345-352), or based on comparisons of Blocks of sequences derived from the Blocks database as described by Henikoff and Henikoff (1992) Proc Natl Acad Sci U S A 89(22):10915-9.

Enamel matrix proteins and/or enamel matrix derivative (EMD) proteins may be in a substantially isolated form. It will be understood that they may be mixed with suitable carriers or diluents, which will not interfere with their intended purpose still be regarded as substantially isolated.

In the present invention, a local algorithm program is best suited to determine identity. Local algorithm programs, (such as Smith-Waterman) compare a subsequence in one sequence with a subsequence in a second sequence, and find the combination of subsequences and the alignment of those subsequences, which yields the highest overall similarity score. Internal gaps, if allowed, are penalized. Local algorithms work well for comparing two multidomain proteins, which have a single domain or just a binding site in common.

Methods to determine identity and similarity are codified in publicly available programs. Preferred computer program methods to determine identity and similarity between two sequences include, but are not limited to, the GCG program package (Devereux, J et al (1994)) BLASTP, BLASTN, and FASTA (Altschul, S.F. et al (1990)). The BLASTX program is publicly available from NCBI and other sources (BLAST Manual, Altschul, S.F. et al (1990)). Each sequence analysis program has a default scoring matrix and default gap penalties. In general, a molecular biologist would be expected to use the default settings established by the software program used.

The proteins of an enamel matrix can typically be divided into a high molecular weight part and a low molecular weight part, which fraction contains acetic acid extractable proteins generally referred to as amelogenins (cf. EP-B-0 337 967 and EP-B-0 263 086).

In general, the enamel matrix, enamel matrix derivatives and enamel matrix proteins are hydrophobic substances, i.e. less soluble in water, especially at increased temperatures. In general, these proteins are soluble at non-physiological pH values and at a low temperature such as about 4-20°C, typically around 4-8°C, while they will aggregate and precipitate at body temperature (35-37°C) and neutral pH (approximately at pH7).

In a specifically preferred embodiment, a formulation for use according to the present invention, thus comprises enamel matrix proteins and/or enamel matrix derivative (EMD) proteins which after application *in vivo* are capable of forming aggregates or oligomeres and to precipitate. The particle size of said aggregates prone to precipitate are less than 70nm, typically in a range between 1nm and 70nm (amelogenin nanospheres) such as from about $1\mu$m to about $20$nm, such as between $1\mu$m and $20$nm, $1\mu$m and $10$nm, $5\mu$m and $10$nm, $10\mu$m and $1$nm, $100\mu$m and $10$nm, $100\mu$m and $1$nm, $1\mu$m and $1$nm, $1\mu$m and $5$nm, $1\mu$m and $15$nm.

**Pharmaceutical formulations**

Pharmaceutical formulations according to the present invention comprise a suitable pharmaceutical carrier selected from the group consisting of a universal buffer, such as a Britton-Robinson buffer, a Carmody buffer, an acetic buffer (acetic acid/sodium acetate), a saline buffer, a citric buffer (citric acid/sodium citrate and/or HCl/sodium citrate), or a physical sodium buffer, aqueous acetic acid, citric acid, and $H_2O$.

If arginine is added to the formulation, it is added to the buffer and pH is adjusted before mixing the buffer with EMD.

**Britton-Robinson buffer** (aka BRB aka PEM) is a "universal" pH buffer used for the range pH 2 to pH 12. Typically, the pH is adjusted to higher values by using NaOH. Universal buffers consist of mixtures of acids of diminishing strength (increasing $pK_a$) so that the change in pH is approximately proportional to the amount of alkali added. It consists of a mixture of 0.04 M $H_3BO_3$, 0.04 M $H_3PO_4$ and 0.04 M $CH_3COOH$ that has been titrated to the desired pH with NaOH. Britton and Robinson also proposed a second formulation that gave an essentially linear pH response to added alkali from pH 2.5 to pH 9.2 (and buffers to pH 12). This mixture consists of 0.0286 M citric acid, 0.0286 M $KH_2PO_4$, 0.0286 M $H_3BO_3$, 0.0286 M veronal and 0.0286 M HCl titrated with 0.2 M NaOH.

In one embodiment, citric acid is used for a formulation with a pH around 2 and TrisHCl for a formulation with a pH around 8.

In the context of the present invention, a formulation may be a pharmaceutical and/or a therapeutic and/or a cosmetic formulation. Presently, a pharmaceutical and/or therapeutic formulation is also intended to embrace cosmetic formulations as well as formulations belonging to the so-called grey area between pharmaceuticals and cosmetics, namely cosmeceuticals.

For the administration to an individual (such as an animal or a human), the enamel matrix proteins and/or enamel matrix derivative (EMD) proteins are preferably formulated into a pharmaceutical formulation containing the enamel matrix proteins and/or enamel matrix derivatives, and, optionally, one or more suitable pharmaceutical carriers.

A formulation comprising the enamel matrix proteins and/or enamel matrix derivative (EMD) proteins according to the present invention, to be administered, may be adapted for administration by any suitable route, e.g. by systemic administration to a patient through a hose, syringe, spray or draining device.

Furthermore, a formulation may be adapted to administration in connection with surgery, e.g. as a systemic administration by infusion into the blood, lymph, ascites, or spinal fluids, or by inhalation. For systemic application, the formulations according to the invention may contain conventionally non-toxic pharmaceutically acceptable carriers and excipients according to the invention, including microspheres and liposomes.

Administration of a formulation according to the present invention may also be performed via any other conventional administration route, such as, but not limited to, an oral, parenteral, intravenous, buccal, aural, rectal, vaginal, intraperitoneal, topical (dermal), or nasal route, or by the administration to a body cavity such as e.g. a tooth root or a tooth root canal.

Other applications may of course also be relevant such as, e. g., application on dentures, protheses, implants, and application to body cavities such as the oral, nasal and vaginal cavity. The mucosa may be selected from oral, buccal, nasal, aural, rectal and vaginal mucosa. Furthermore, the application may be directly on or onto a wound or other soft tissue injuries.

Furthermore, application within the dental/odontologic area is of great importance. Relevant examples are application to periodontal (dental) pockets, to gingiva or to gingival wounds or other wounds located in the oral cavity, or in connection with oral surgery.

A formulation for use in accordance with the present invention may be, but is not limited to, in the form of, e.g., a fluid, semi-solid or solid formulation such as, but not limited to, dissolved transfusion liquids, such as sterile saline, Ringer's solution, glucose solutions, phosphate buffer saline, blood, plasma, water, powders, microcapsules, bioabsorbable patches, drenches, sheets, bandages, plasters, implants, pills, sprays, soaps, suppositories, vagitories, toothpaste, lotions, mouthwash, shampoo, microspheres, nanoparticles, sprays, aerosols, inhalation devices, solutions, dispersions, wetting agents, suspensions, emulsions, pastes, ointments, hydrophilic ointments, creams, gels, hydrogels (e.g. poly ethylene glycols), dressings, devices, templates, smart gels, grafts, solutions, emulsions, suspensions, powders, films, foams, pads, sponges (e.g. collagen sponges), transdermal delivery systems, granules, granulates, capsules, agarose or chitosan beads, tablets, microcapsules, freeze-dried powders, granules, granulates or pellets, and mixtures thereof.

Suitable suspending agents are, e.g., naturally occurring gums such as, e.g., gum acacia, xanthan gum, or gum tragacanth; celluloses such as, e.g., sodium carboxymethylcellulose, microcrystalline cellulose (e.g. Avicel® RC 591, methylcellulose); alginates and kitosans such as, but not limited to, sodium alginate, etc.

A liquid formulation, for use in accordance with the present invention, may e.g. be, but is not limited to, a solution, dispersion or suspension for application on a surface of e.g. a medical implant or device, or bone replacement material. Once applied, the formulation can solidify, e.g. by drying, to a solid or at least highly viscous formulation which does not dissolve on storage or when the implant or device is in use.

Such a formulation is preferably applied under sterile conditions and/or sterilised after application by irradiation or exposure to ethylene oxide gas. When the formulation is in the form of a liquid formulation, it may also be applied shortly before the medical implant or device, or bone replacement material is to be introduced into the body. As an alternative to applying a formulation comprising enamel matrix proteins and/or enamel matrix derivative (EMD) proteins on the medical implant or device, or bone replacement material, the formulation may be applied on a surface of a tissue which is in contact with the medical implant or device, or bone replacement material, such as a tissue comprising a substantial proportion of epithelial cells as indicated above. Furthermore, the formulation may be applied on both the medical implant or device, or bone replacement material and on a tissue in contact therewith.

A formulation according to the present invention, may also, in addition to what already has been disclosed herein, be formulated according to conventional pharmaceutical practice, see, e.g., "Remington's Pharmaceutical Sciences" and "Encyclopedia of Pharmaceutical Technology", edited by Swarbrick, J. & J. C. Boylan, Marcel Dekker, Inc., New York, 1988.

A suitable pharmaceutical carrier is a substance, which is substantially harmless to the individual to which the formulation is to be administered. Such an excipient normally fulfils the requirements given by the national health authorities. Official pharmacopoeias such as e.g. the British Pharmacopoeia, the United States of America Pharmacopoeia and The European Pharmacopoeia set standards for suitable pharmaceutical carriers.

The suitable pharmaceutical carriers may include solvents, buffering agents, preservatives, humectants, chelating agents, antioxidants, stabilizers, emulsifying agents, suspending agents, gel-forming agents, ointment bases, penetration enhancers, perfumes, powders and skin protective agents. It should however be emphasized that the invention is not limited thereto.

In the present invention, the formulation comprising the enamel matrix proteins and/or enamel matrix derivative (EMD) proteins, can be incorporated into a polymeric matrix so that it is released by degradation of the polymeric matrix, by enzymatic action and/or by diffusion. Said polymeric matrix is either suitable for cellular in-growth, or cell-occlusive. Comprised in the invention is thus in particular a pharmaceutical and/or cosmetic formulation of the enamel matrix proteins and/or enamel matrix derivative (EMD) proteins, at a low total concentration within the formulation, wherein a spatial and/or selective regulation of release of said enamel matrix proteins and/or enamel matrix derivative (EMD) proteins permits a great percentage of the enamel matrix proteins and/or enamel matrix derivative (EMD) proteins to be released at the time of appropriate cellular activity.

Consequently, one aspect of the present invention relates to a pharmaceutical and/or therapeutic formulation for administering the enamel matrix proteins and/or enamel matrix derivative (EMD) proteins according to the present invention, comprising a polymeric matrix, either suitable for cellular growth, in-growth and/or migration, or being cell-occlusive, and a fraction and/or polypeptide fragment, wherein said matrix is formed by a nucleophilic addition reaction between a strong nucleophile and a conjugated unsaturated bond, or a conjugated unsaturated group.

In a toothpaste or mouthwash formulation or other formulation for application to teeth or tooth roots, enamel matrix proteins and/or enamel matrix derivative (EMD) proteins according to the present invention may either be present in a dissolved state in a vehicle of slightly acid pH or as a dispersion in a vehicle of neutral pH. It is anticipated that enamel matrix proteins and/or enamel matrix derivative (EMD) proteins according to the present invention may form a protective layer on the surface of the teeth, thereby preventing the attachment of caries producing bacteria. In such dental care preparations, enamel matrix proteins and/or enamel matrix derivative (EMD) proteins may be formulated together with one or more other compounds which have a caries preventive effect, notably fluorine or another trace element such as vanadium or molybdenum. At neutral pH, the trace element is believed to be bound to (e. g. by ion bonds) or embedded in the enamel matrix proteins and/or enamel matrix derivative (EMD) proteins from which it is released to exert its caries preventive effect when the enamel matrix proteins and/or enamel matrix derivative (EMD) proteins is dissolved at a pH of about 5.5 or less, e. g. due to acid production by caries producing bacteria.

In a pharmaceutical formulation for use according to the invention, the enamel matrix proteins and/or enamel matrix derivatives according to the present invention are generally present in a concentration ranging from about 0.01% to about 99.9% w/w. The amount of formulation applied will normally result in an amount of total protein per $cm^2$ area of dental pulp corresponding to from about 0.005 $mg/mm^2$ to about 5 $mg/mm^2$ such as from about 0.01 $mg/mm^2$ to about 3 $mg/mm^2$.

In those cases where the enamel matrix proteins and/or enamel matrix derivatives according to the present invention are administered in the form of a liquid formulation, the concentration of the enamel matrix proteins and/or enamel matrix derivatives according to the present invention in the formulation is in a range corresponding to from about 0.01 to about 50 mg/ml, e.g. from about 0.1 to about 35 mg/ml, such as 30 mg/ml. Higher concentrations are in some cases desirable and can also be obtained such as a concentration of at least about 100 mg/ml.

**LEGENDS TO FIGURES**

Fig 1. UV-Vis absorption of EMD in BR buffer at pH2 during a temperaturerise from 20 to 30 °C and at higher temperatures. In presence of arginine (Fig. 1(b)). Results obtained at pH5 are shown in Figs. 1(c) and 1(d).

Fig 2. Second derivative FT-IR spectra of EMD (A) and EMD/arginine (B) at pH 2 and 25 °C, before (solid curves) and after (hatched curves) heat treatment at 90 °C. 3D-DLS spectra EMD (C) and EMD/arginine (D) at pH 2 and 20 °C, before (blue) and after (red) heat treatment at 70 °C.

Fig 3. Transmission electron micrographs of EMD and EMD/arginine at pH 2 and 20 °C before (A-C) and after (B-D) heat treatment at 90 °C.

Fig 4. Second derivative FT-IR spectra of EMD (A) and EMD/arginine (B) at pH 5 and 25 °C, before (solid curves) and after (hatched curves) heat treatment at 90 °C. 3D-DLS spectra EMD (C) and EMD/arginine (D) at pH 5 and 20 °C, before (blue) and after (red) heat treatment at 70 °C.

Fig 5. Transmission electron micrographs of EMD and EMD/arginine at 20 °C and pH 5 before (A-C) and after (B-D) heat treatment at 90 °C.

Fig 6. Second derivative FT-IR spectra of EMD (A) and EMD/arginine (B) at pH 10 and 25 °C, before (solid curves) and after (hatched curves) heat treatment at 90 °C. 3D-DLS spectra EMD (C) and EMD/arginine (D) at pH 10 and 20 °C, before (blue) and after (red) heat treatment at 70 °C.

Fig 7. Transmission electron micrographs of EMD and EMD/arginine at pH 10 and 20 °C before (A-C) and after (B-D) heat treatment at 90 °C.

Fig 8. T(t) from stopped flow measurements on (A) EMD and (B) PGA/EMD in BR-buffer (pH2 or pH5), with or without arginine, mixed with sodium hydroxide to raise its pH to 7.

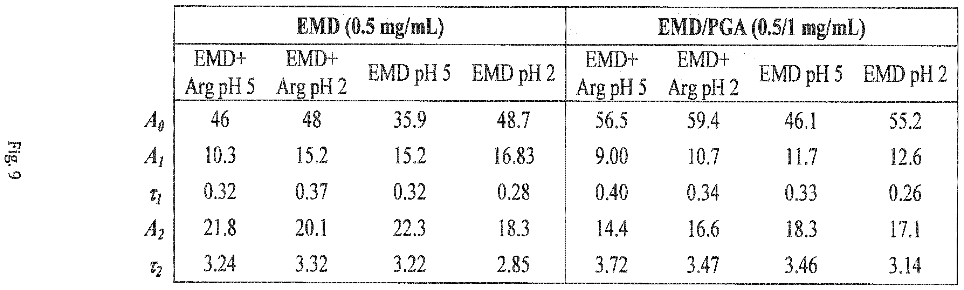

Fig 9. Table 1. Summary of parameters used to fit equation (1) to the data in Fig 8.

Fig. 10 shows the transient precipitation kinetics from aqueous EMD and Straumann Emdogain™ (EMD/PGA) gel, as reflected by the stopped flow measurements of the transmittance, $T$, as a function of time, $t$, after raising the pH of the specimens to 7 i.e. close to the EMD isoelectric point (pH 6.8).

Fig 11. Experimental configuration for the stopped-flow measurements, comprising a BioLogic SFM-400 stopped-flow photospectrometer equipped with four syringes, a Berger ball mixer (BB) and a high density mixer (HDS) (29).

## EXPERIMENTAL SECTION

The presented experiments describe efforts to improve the stability and hence prolong the regenerative capacity of the Emdogain™ gel during storage at low pH, through use of low pH and/or arginine as an aggregation suppressor. EMD specimens with and without low pH and/or arginine were analyzed by Fourier transform infrared spectroscopy, UV-Vis absorbance spectroscopy, 3D-dynamic light scattering and transmission electron microscopy. The results demonstrated that arginine not only stabilizes the native structure of the EMD during thermal cycling at low pH, but may also induce refolding at high pH. Moreover, stopped flow photospectrometry indicated the presence of arginine not to influence precipitation of EMD from either aqueous solution or the gel complex subsequent to a step-like increase in pH.

**Experiment 1:**

**Materials and methods**

*EMD*

2012 EMD solution (Biora/Straumann) with a concentration of 29 mg/ml in 10 mg/ml aqueous acetic acid was lyophilized using a Vitris freeze dryer, operating at -8°C. 100 to 150 g of the EMD solution was placed in two 250 ml conical flasks, which were immersed in liquid nitrogen and lyophilized over 48 h. The lyophilized material was collected and homogenized by gentle mixing with a spatula, mortar and pestle. The EMD was then redissolved at room temperature (RT) in Britton-Robinson buffer (0.1M of pure $H_3BO_3$, $H_3PO_4$, $CH_3COOH$), and the pH adjusted to 2, 2.5 and 10 at an EMD concentration of 29 mg/ml. Arginine with a concentration of 500mM was added to buffer solutions before the addition of EMD. Specimens with and without arginine were stored at 4 °C overnight prior to characterization in order to stabilize them.

*PGA*

The PGA originated from FMC BioPolymer, and was sterilized by Sterigenics, San Diego using a 20 eV electron accelerator (30 kGy per 100 g pouch). It was provided by Biora AB (Straumann AG, Basel) and stored at 4 °C in a refrigerator prior to use.
Protein-carrier (EMD/PGA) hydrocolloid complexes were prepared by adding aqueous EMD to PGA powder (EMD/PGA 1:2). The mixtures were stirred with a magnetic stirring bar until the polymer fully dissolved at room temperature and were subsequently stored at 4 °C overnight for stabilization. All specimens were prepared using double deionized milli-Q water.

*Transmission mode Fourier Transform Infrared spectroscopy (FT-IR)*

Infrared spectroscopy in transmission mode was used to monitor changes in the secondary structure of the EMD after heat treatment (17). 200 $\mu$l aliquots of EMD solutions prepared with different pH were incubated at 25 °C for 1 hour. The temperature was then increased to 90 °C at 2 °C/min and the aliquots incubated at this temperature for a further 1 hour. They were then cooled to 25°C at 2 °C/min and incubated for 12 hours at this temperature to improve their stability. IR absorbance measurements were carried out before and after the heat treatment by depositing a 10 $\mu$l drop of each specimen onto a polished $CaF_2$ IR window and letting it dry. After acquisition, secondary derivative analysis and normalization were used to resolve the fine structure of the spectra in the amide I region.

*UV-Vis absorption spectroscopy*

Aqueous EMD was placed in a 1mm quartz cuvette and analyzed by a V-670 Varian spectrometer at a fixed wavelength of 280 nm. This value corresponds to the absorption of tryptophan, an amino acid present in the hydrophobic core of proteins. The absorption intensity, which reflects EMD solubility, i.e. the exposure of tryptophan to the solvent, was measured at temperatures between 20 and 80 °C (Schmid, 2001).

*Dynamic Light Scattering*

Advanced modulated 3D cross correlation dynamic light scattering (DLS) technology was used to investigate the EMD aggregate dimensions. The measurements were performed using an LS Instruments apparatus equipped with a He-Ne laser emitting a polarized light beam of wavelength of 632.8 nm. Specimens were placed in 10 mm diameter glass tubes, and measurements performed at temperatures from 20 to 70 °C in steps of 10 °C. The sample cell was maintained at within +/- 0.1 °C of the required temperature using a program-controlled thermostat. The scattered intensity fluctuations were collected at a fixed angle of 90 ° by averaging 3 runs of 600 seconds each. The time correlation function (TCF) of the scattered intensity was analyzed using the CONTIN method (Block, I. D. & Scheffold, F. Modulated 3D cross-correlation light scattering: Improving turbid sample characterization. Review of Scientific Instruments, 2010; 81 : 123107-7.).

*Transmission Electron Microscopy (TEM)*

The morphology of the EMD with and without arginine, in different buffers and at different pH, was investigated using a 80 kV Tecnai Spirit BioTWIN Transmission Electron Microscope (TEM) in bright field mode. Selected specimens were heat treated at 90 °C, cooled to 25 °C and incubated for 12 h. Specimens for TEM analysis were prepared using negative staining by a two-droplet sequential method as described in the literature (http://web.path.ox.ac.uk/~bioimaging/bitm/instructions_ and_information/EM/neg_stain.pdf).

*Stopped flow photospectrometry*

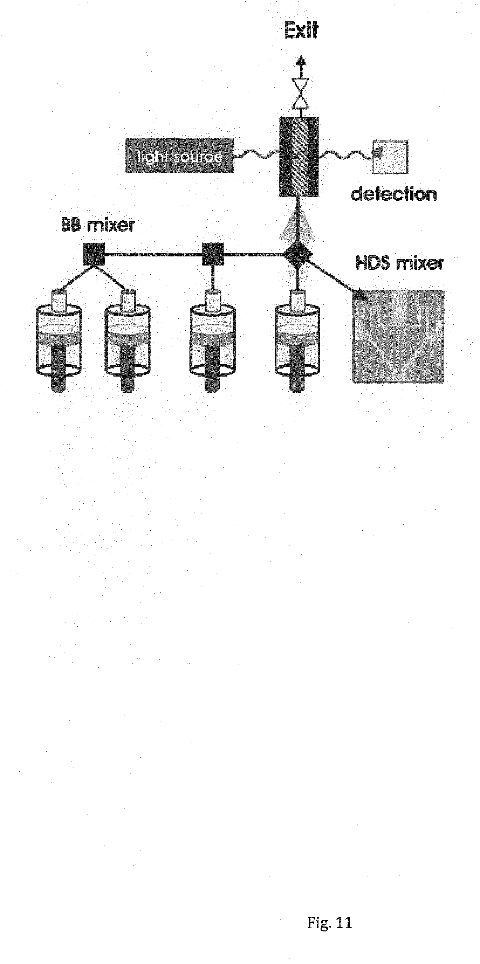

The release kinetics from aqueous EMD and EMD/PGA, and their arginine-stabilized counterparts were investigated by rapid kinetics stopped flow (SF) photospectrometry, with a BioLogic SFM400/S device connected to a MOS-250 spectrometer unit, emitting light at a wavelength of 290 nm. 1 mg/ml EMD specimens with and without arginine were prepared in Britton-Robinson buffer at pH 2 and 5. The pH was then increased to 7 (i.e. physiological pH) by addition of 250 mM sodium hydroxide (NaOH) to the specimens initially at pH 2 and 50 mM NaOH to the specimens initially at pH 5. A total flow rate of 6 ml/s and total flow cell volume of approximately 351 $\mu$l were used throughout. Under these conditions use of a FC-15 flow cell for the transmittance detection implied a dead time of 10 ms. The buffered specimens and NaOH solutions were stored in high performance syringes and equivalent volumes injected (1:1) via a high-density mixer into the observation flow cell (1.5 mm light path length). The specimen transmittance was measured over a time of approximately 15 s and reproducibility verified by repeating the measurements four times. The method and experimental set-up are shown schematically in Fig 11 (Barth, I. Grillo, and M. Gradzielski. Dynamics of formation of vesicles studied by highly time-resolved stopped-flow experiments. Tenside Surf. Det., 2010, 47:300-306).

**Results and discussion**

*Structural analysis of EMD solutions with and without arginine at different pH*

It is well known in the field of the art that EMD is most soluble at 4°C. Therefore, EMD was dissolved at 4°C and then analyzed at 25 °C (RT), before and after thermal treatment at T=80, 70 and 100°C , respectively, in order to investigate its stability around RT even after a strong temperature stress.

*Conformation and aggregation behavior of EMD*

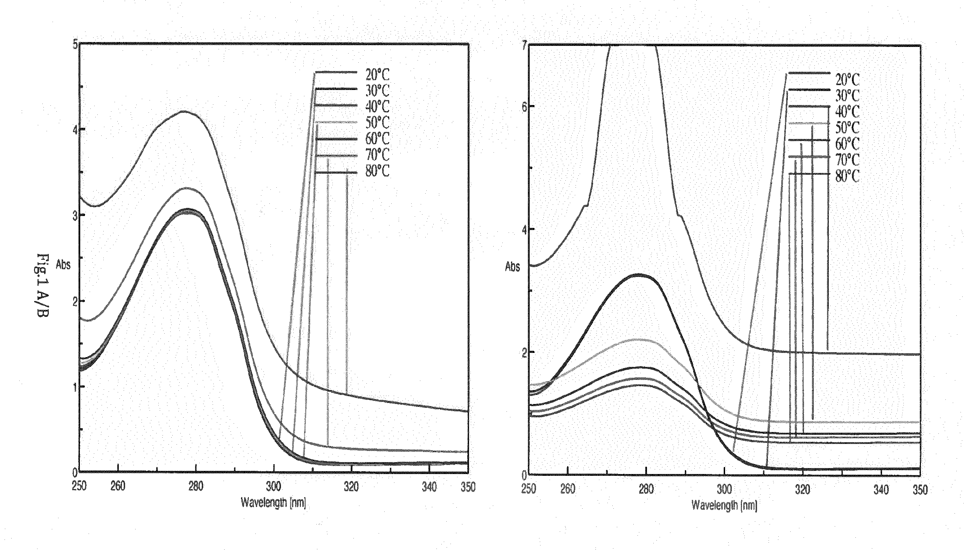

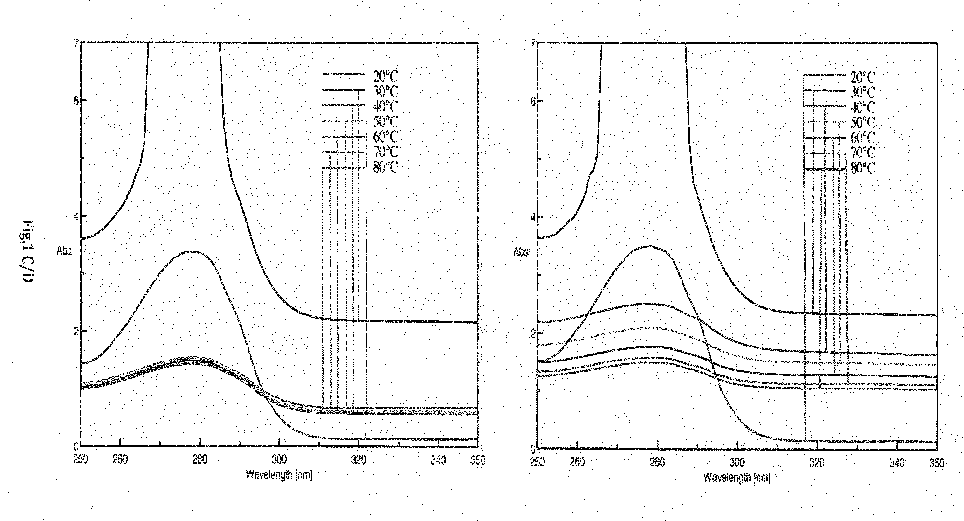

As shown in Fig. 1(a), the UV-Vis absorption of EMD in BR buffer at pH2 increased when the temperature was raised from 20 to 30 °C, indicating a tendency for the EMD proteins to unfold, exposing their inner residues to the buffer. The trend was reversed at higher temperatures, however, the absorption gradually decreasing from its initial level, implying EMD to begin to precipitate from around 40 °C. Two main differences were observed in presence of arginine (Fig. 1(b)): the baseline intensity at 20 °C increased, indicating more extensive aggregation at low temperatures than in the absence of arginine, and the decrease in absorption at high temperatures was less marked, suggesting a reduced precipitation rate. Results obtained at pH5 are shown in Figs. 1(c) and 1(d). The behavior was qualitatively similar to that observed at pH2, but the absorption reached saturation at 30 °C in this case, reflecting an increased tendency for EMD to solubilize at pH5, even in the presence of arginine, although arginine was again inferred to cause a decrease in precipitation rates at high temperatures.

Fig 2A shows second derivative FT-IR spectra of EMD in Britton-Robinson buffer at pH 2 in the wavenumber range corresponding to the amide I region. At 25 °C, the spectra for the as-prepared EMD specimens showed peaks at 1652 and 1660 cm$^{-1}$, which were assigned to the $\alpha$-helices. Native $\beta$-sheets peaks were also visible at 1620, 1633 and 1643 cm$^{-1}$ as well as an intermolecular $\beta$-sheets peak at 1698 cm$^{-1}$ (Lambright DG, Balasubramanian S, Boxer SG. Protein relaxation dynamics in human myoglobin. Chemical Physics, 1991. 158:249-260.). After heat treatment and incubation for 12 h at 25 °C, the intensity of the peak at 1660 cm$^{-1}$ decreased and the peak at 1652 cm$^{-1}$ was replaced by a peak at 1654 cm$^{-1}$, which has been previously linked to disordered conformations (Barth A, Zscherp C. What vibrations tell us about proteins. Quarterly Reviews of Biophysics, 2002 ; 35(4):369-430.), suggesting significant conformational changes of the $\alpha$-helices. While the native $\beta$-sheets peak at 1633 cm$^{-1}$ was not greatly affected by the heat treatment, the remaining peaks showed modified intensities and positions. Thus, the peak at 1620 cm$^{-1}$ was replaced by a more intense peak at 1617 cm$^{-1}$, the intermolecular $\beta$-sheets peak at 1698 cm$^{-1}$ was shifted to about 1697 cm$^{-1}$ and an additional peak appeared at 1688 cm$^{-1}$. This is significant in so far as intense peaks in the 1620-1615 cm$^{-1}$ range and at the high wavenumber limit of the amide I region may indicate exposition of the intermolecular contacts (Barth A, Zscherp C. What vibrations tell us about proteins. Quarterly Reviews of Biophysics, 2002 ; 35(4):369-430.). Three further peaks at 1668, 1675 and 1682 cm$^{-1}$ were assigned to $\beta$-turns, which are considered to be indicators of the compactness of protein structures and may be associated with aggregation sites because they also appear at the external surfaces of proteins. After heat treatment, the main $\beta$-turns peak at 1682 cm$^{-1}$ shifted to about 1680 cm$^{-1}$, again possibly indicating aggregation (Barth A, Zscherp C. What vibrations tell us about proteins. Quarterly Reviews of Biophysics, 2002; 35(4):369-430.). Heat treatment also resulted in a slight increase in the intensity of the $\beta$-turns peak at 1668 and a more substantial decrease in the intensity of the peak at 1675 cm$^{-1}$.

Addition of arginine at pH 2 led to a decrease in the intensity of the $\alpha$-helices peak centered at around 1660 cm$^{-1}$ prior to heat treatment, and a further small decrease after heat treatment, while the peak at 1652 cm$^{-1}$ was present both before and after heat treatment (Fig 2B), indicating the conformations associated with the $\alpha$-helices to remain stable. Moreover, the $\beta$-sheets peak at about 1620 cm$^{-1}$ remained unchanged after heat treatment, which suggests limited conformational change of the exposed surface to have taken place. The most intense peak in the spectrum before and after heat treatment was the $\beta$-turns peak at about 1681 cm$^{-1}$. The position of this peak was the same as that observed after heat treatment in the absence of arginine, in which case the shift from its original value of 1682 cm$^{-1}$ to 1681 cm$^{-1}$ was attributed to aggregation. This suggests that protein-protein interaction is not prevented by the presence of arginine, which is expected to promote compactness of the protein structure, accounting for the high intensity of the $\beta$-turn peak at 1681 cm$^{-1}$. Taken together, these results imply arginine to stabilize the EMD structure at pH 2 with respect to heat treatment. However, it is not clear whether arginine prevents heat-induced unfolding and/or denaturation, or whether it merely favors refolding at 25 °C subsequent to denaturation at high temperatures.
To investigate this further, 3D-DLS and UV-Vis absorbance experiments were therefore performed *in situ.*

The EMD particle size distribution in Britton Robinson buffer at pH 2 in the absence of arginine showed two main peaks at approximately 8 and 80 nm prior to heat treatment, as determined by DLS (Fig 2C). After heat treatment up to 70 °C and cooling to 20 °C the size distribution became far broader with peaks appearing at higher values. Thus, consistent with the FT-IR results, conformational changes dominated in this case, as also reflected by the TEM micrographs in Fig 3A-B. Nanofilaments and nanospheres of about 70 nm or less in diameter, typical of amelogenin in its native state (Fanga PA, Conwayb JF, Margolisc HC, Simmerd JP, Beniasha E. Hierarchical self-assembly of amelogenin and the regulation of biomineralization at the nanoscale. Pnas 2011 ; 108(34) :14097-14102.), are visible in Fig 3A, but, as seen in Fig 3B, these were partially precipitated after heat treatment. The presence of arginine at pH 2 conferes compactness to EMD, as suggested by Fig 2D which was also consistent with the FT-IR results. Since FT-IR showed denaturation of the EMD not to occur in presence of arginine, it is concluded that heat-induced unfolding was absent at pH 2. Hence, the nanosphere structure of the EMD remained substantially intact (Fig 3C-D).

Moreover, while the heat induced conformational changes associated with the peak at 330 nm were still present, they were less marked. Indeed, the initial state of the specimen was largely recovered after subsequent cooling and incubation at 20 °C, as reflected by the form of the absorbance curves at low wavelengths, providing further evidence that arginine limits structural rearrangements, presumably by binding to the hydrophobic regions of the constituent proteins of the EMD.

At pH 5 the FT-IR spectrum of the as-prepared EMD (Fig 2A) showed a particularly strong $\alpha$-helices peak at 1652 cm$^{-1}$ while the intensity of the $\alpha$-helices peak at 1660 cm$^{-1}$ was comparable with that observed at pH 2 (Fig 2A). At lower wavenumbers, peaks characteristic of native $\beta$-sheets were observed at 1633, 1623 and 1615 cm$^{-1}$, this latter peak being absent at pH 2 (Fig 2A). $\beta$-turn peaks were also present at 1668 and 1682 cm$^{-1}$, but the peak at around 1675 cm$^{-1}$ mergered with the main peak at 163 cm$^{-1}$ and was far weaker than at pH 2, suggesting a less compact structure. Finally, the low intensities of the peaks in the high wavenumber region of the spectrum at around 1688 cm$^{-1}$ and 1695 cm$^{-1}$, corresponding to the intermolecular $\beta$-sheet band, indicated relatively weak intermolecular contacts. Heat treatment resulted in some changes in the intensity of these peaks, but no major qualitative modification to the secondary structure, with the exception of the native and intermolecular $\beta$-sheet peaks at 1643 cm$^{-1}$ and 1695 cm$^{-1}$, respectively, which shifted to lower wavenumbers. Hence, while the molecular organization of the EMD was significantly modified by increasing the pH, it did not undergo further major changes after heat treatment.

The main consequence of addition of arginine to EMD at pH 5 was a change in the conformations corresponding to the high wavenumber region, i.e. in the $\beta$-turns and intermolecular $\beta$-sheets bands (Fig 4B). Prior to heat treatment, the $\beta$-turn peak observed at around 1675 cm$^{-1}$ in the absence of arginine merged with the main peak centered on 1668 cm$^{-1}$, and the intermolecular $\beta$-sheets peak at 1695 cm$^{-1}$ increased in intensity. In the presence of arginine, both the $\beta$-turns components were distinguishable. Without wishing to limit the invention to this particular hypothesis, this effect may be attributed to the formation of complexes between the arginine and ions in the buffert which exclude proteins from interacting with the solvent. Further, arginine may also interact with the external binding sites of the EMD. Heat treatment resulted in a modification of the $\beta$-turns, as reflected by the reappearance of two peaks at 1668 cm$^{-1}$ and 1675 cm$^{-1}$, and the intermolecular $\beta$-sheet peaks at high wavenumbers decreased in intensity. However, while heat treatment in the presence of arginine affected the peaks associated with intermolecular interactions, the native $\alpha$-helices (1652 and 1660 cm$^{-1}$) appeared more stable than in absence of arginine, where the corresponding peaks showed significant changes in intensity.

The DLS results in Fig 4C showed the maximum of particle size distribution of the as-prepared EMD at pH 5 to be at about 80 nm. Heat treatment led to increased aggregation, as indicated by the broadening of the peak and a shift towards 500 nm, and also by the TEM images (Fig 5A-B). Nanofilaments and isolated nanospheres were initially present (Fig 5A), but extensive agglomeration was observed after heat treatment (Fig 5B). Given that FT-IR indicated little unfolding, the nanospheres, whose dimensions in the as-prepared EMD were again consistent with those typical of amelogenin (Fanga PA, Conwayb JF, Margolisc HC, Simmerd JP, Beniasha E. Hierarchical self- assembly of amelogenin and the regulation of biomineralization at the nanoscale. Pnas 2011 ; 108(34) :14097-14102.), were assumed to aggregate during heat treatment. As seen from Fig 4D, arginine limited this aggregation. Indeed, after heat treatment in the presence of arginine the main DLS peak at about 100 nm shifted to below 80 nm and decreased slightly in intensity, and an additional peak appeared at approximately 6 nm. Hence, as observed at pH 2, heat treatment may have resulted in precipitation of bigger aggregates. This was reflected by the TEM images in Fig 5C-D, which suggest initial nanofilaments present in the specimens to have aggregated and precipitated under and/or after heat treatment, so that only nanospheres were imaged.

At pH 10, the protein conformations were strongly modified with respect the native conformations seen at lower pH, as borne out by the FTIR spectra (Fig 6A), which indicated a relatively high proportion of random coil structure for example. Moreover, the combination of high pH and ionic charges stabilized the secondary structure during heat treatment in the absence of arginine. In the presence of arginine, on the other hand, the spectra obtained before and after heat treatment no longer overlapped, the proportion of random coils was reduced, and peaks corresponding to the $\beta$-turn structures were again distinguishable (Fig 6B).

While the DLS results (Fig 6C-D) provided little additional insight, the TEM micrographs in Fig 7 confirmed the morphology of the EMD to be very different to that observed at lower pH, being characterized by extensive amorphous gel-like agglomerates.

*Release kinetics from EMD solutions and EMD/PGA gels: the role of arginine.*

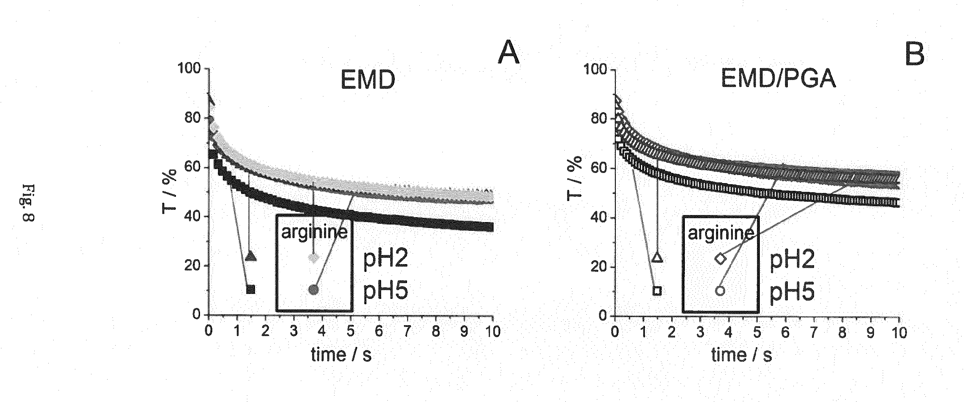

Fig 8 shows the transient release kinetics from aqueous EMD and EMD/PGA, as reflected by the stopped flow measurements of the transmittance, $T$, as a function of time, $t$, after raising the pH of the specimens to 7 i.e. close to the EMD isoelectric point (pH 6.8). These results again showed reduction of the initial pH to 2 or addition of arginine to result in stabilization of the native EMD conformations. This was apparent for both the EMD and the EMD/PGA gels, in so far as the respective data sets superimposed, indicating similar release dynamics, with the exception of the specimens prepared without arginine at pH5 (Fig 8A-B), which showed significantly greater turbidity (i.e. lower $T$) at a given $t$, and hence a greater tendency to form large precipitates. It follows that complexation with PGA did not modify the intrinsic response of the EMD. Comparison of Fig 8A and Fig 8B nevertheless suggests the PGA to limit precipitation of the EMD, $T$ being some 10 % greater for the EMD/PGA gels than for the EMD at the longest $t$, presumably owing to physical confinement of the EMD by the PGA matrix. This implies full precipitation of the EMD only to be possible if the PGA is at least partly dissolved by the solvent medium. The effect of the PGA could be further demonstrated by fitting the data for $T(t)$ using a second order exponential decay function:

$$T(t) = A_0 + A_1 \exp\left(-\frac{t}{\tau_1}\right) + A_2 \exp\left(-\frac{t}{\tau_2}\right)$$

. . . Eq. (1).T

Eq. 1 describes two stages in the formation of protein precipitates, a fast process with amplitude $A_1$ and decay constant $T_1$, and a slow gravity dependent process with amplitude $A_2$ and decay constant $T_2$ As shown in Table 1, the decay constants for the slow process were significantly greater for EMD/PGA than for EMD.

These results indicate addition of arginine not to have an adverse influence on the precipitation under conditions representative for its application in surgical procedures, and confirm the stabilizing effect of the arginine molecules on EMD with respect to aggregation at pH 5 in the presence of PGA. Moreover, the PGA has no such stabilizing effect, so that the slower effective release rates from the EMD/PGA gels may be attributed to physical entrapment of the EMD in the PGA hydrogel network, retarding large-scale agglomeration during the later stages of precipitation.

*Precipitation kinetics from EMD solutions and EMD/PGA gels: the role of arginine.*

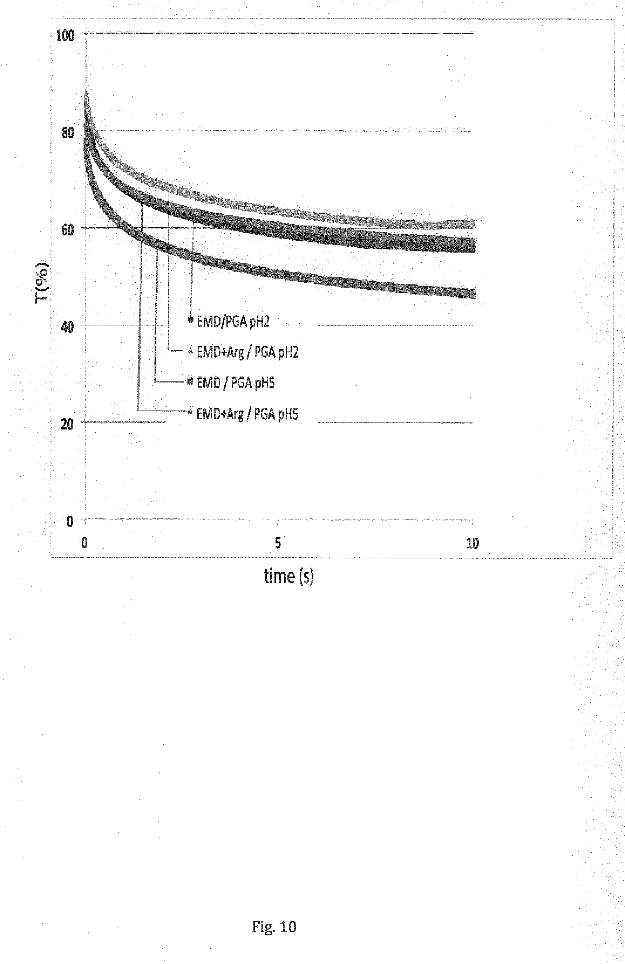

Fig. 10 shows the transient precipitation kinetics from aqueous EMD and Straumann Emdogain™ (EMD/PGA) gel, as reflected by the stopped flow measurements of the transmittance, $T$, as a function of time, $t$, after raising the pH of the specimens to 7 i.e. close to the EMD isoelectric point (pH 6.8). These results again showed reduction of the initial pH to 2 or addition of arginine to result in stabilization of EMD conformations. This was apparent for both the EMD and the EMD/PGA gels, in so far as the respective data sets superimposed, indicating similar release dynamics, with the exception of the specimens prepared without arginine at pH5 (Fig. 10(a)-(b) and Table 1), which showed significantly greater turbidity (i.e. lower $T$) at a given $t$, and hence a greater tendency to form large precipitates. It follows that formation of complexes with PGA did not modify the intrinsic response of EMD under conditions representative of its application to damaged periodontal tissue.

**Conclusions**

Addition of arginine to aqueous EMD has been shown using a variety of analytical techniques to limit denaturation of the EMD at acidic pH, and there is also evidence that it may reverse large-scale unfolding at high pH. Investigation of the release kinetics of EMD subsequent to a step-like increase in pH to pH 7, which is close the isoelectric point of the EMD, also showed arginine not to have an adverse influence on the precipitation rates from either aqueous solution or from a PGA gel carrier. Indeed, the response in systems with an initial pH of 5 was similar with arginine to that at pH 2 both with and without arginine, again reflecting the stabilizing effect of arginine on the EMD. These results therefore indicate that arginine, whose use in products for dental treatment is already validated (Sakamoto R, Nishikori S, Shiraki K. High temperature increases the refolding yield of reduced lysozyme: implication for the productive process for folding. Biotechonl. Prog 2004; 20:1128-1133.15. Petrou I, Heu R, Stranick M, Lavander S, Zaidel L, Cummins D et al. A breakthrough therapy for dentin hypersensitivity: how dental products containing 8% arginine and calcium carbonate work to deliver effective relief of sensitive teeth. The journal of Clinical Dentistry 2009; 20 (1): 23-31.), has the potential to improve significantly the storage life of EMD/PGA-based gels used in regenerative therapy, without compromising the release of the EMD at the site of application.

**REFERENCES**

1. Gestrelius S, Lyngstadaas SP, Hammarstrøm L. Emdogain - periodontal regeneration based on biomimicry. Clin Oral Invest 4:120-125 (2000).
2. Hammarström et al., 1997, Journal of Clinical Periodontology 24, 658-668
3. Lyngstadaas et al., 2001, Journal of Clinical Periodontology 28, 181-188
4. US Patent No. 4,672,032

5. EP-B-0 337 967

6. EP-B-0 263 086

7. EP-1059934

8. EP- 01201915.4

9. WO 01/97834

10. WO 00/53197

11. WO 00/53196

12. WO 03/024479

13. WO 02/080994

14. Casal HL, Kohler U, Mantsch HH. Structural and conformational changes of β-lactoglobulin B : an infrared spectroscopic study of the effect of pH and temperature. Biochimica et Biohysica Acta (BBA)- Protein structure and Molecular Enzymology 1998 ; 957(1) : 11-20.

15. Lambright DG, Balasubramanian S, Boxer SG. Protein relaxation dynamics in human myoglobin. Chemical Physics, 1991. 158:249-260.

16. Sambrook, J. et al.: Molecular Cloning, Cold Spring Harbor Laboratory Press, 1989.

17. Ten Cate: Oral Histology, 1994; Robinson: Eur. J. Oral Science, Jan. 1998, 106 Suppl. 1:282-91.

18. Dayhoff, Schwartz, and Orcutt (1978) Atlas Protein Seq. Struc. 5:345-352.

19. Henikoff and Henikoff (1992) Proc Natl Acad Sci USA 89(22):10915-9.

20. Devereux, J et al (1994).

21. Altschul, S.F. et al (1990).

22. Remington's Pharmaceutical Sciences

23. Encyclopedia of Pharmaceutical Technology", edited by Swarbrick, J. & J. C. Boylan, Marcel Dekker, Inc., New York, 1988.

24. KONG J,YU S. Fourier Transform Infrared Spectroscopic Analysis of Protein Secondary Structures. Acta Biochimica et Biophysica Sinica 2007; 39 (8): 549-559.

25. Herberhold H, Marchal S, Lange R, Scheyhing CH, Vogeland RF, Winter R. Characterization of the Pressure-induced Intermediate and Unfolded State of Red-shifted Green Fluorescent Protein-A Static and Kinetic FTIR, UV/VIS and Fluorescence Spectroscopy Study. J. Mol. Biol. 2003; 330: 1153-1164.

26. Block, I. D. & Scheffold, F. Modulated 3D cross-correlation light scattering: Improving turbid sample characterization. Review of Scientific Instruments, 2010; 81 : 123107-7.

27. TEM

28. http://web.path.ox.ac.uk/~bioimaging/bitm/instructions and information/EM/neg st ain.pdf.

29. Barth, I. Grillo, and M. Gradzielski. Dynamics of formation of vesicles studied by highly time-resolved stopped-flow experiments. Tenside Surf. Det., 2010, 47:300-306.

30. Barth A, Zscherp C. What vibrations tell us about proteins. Quarterly Reviews of Biophysics, 2002 ; 35(4):369-430.

31. Fanga PA, Conwayb JF, Margolisc HC, Simmerd JP, Beniasha E. Hierarchical self- assembly of amelogenin and the regulation of biomineralization at the nanoscale. Pnas 2011 ; 108(34):14097-14102.

32. Schneider, CP, Shukla, D, Trout BL, Arginine and Hofmeister series, the role of ion-ion interactions in protein aggregation suppressoipn, J. Phys.Chem.B 2011, 115, 7447-7458.

33. Sakamoto R, Nishikori S, Shiraki K. High temperature increases the refolding yield of reduced lysozyme: implication for the productive process for folding. Biotechonl. Prog 2004; 20:1128-1133.15.

34. Petrou I, Heu R, Stranick M, Lavander S, Zaidel L, Cummins D et al. A breakthrough therapy for dentin hypersensitivity: how dental products containing 8% arginine and calcium carbonate work to deliver effective relief of senssitive teeth. The journal of Clinical Dentistry 2009; 20 (1): 23-31.

## Claims

1. A process for producing a pharmaceutical, dental and/or cosmetic formulation comprising enamel matrix proteins and/or enamel matrix derivative (EMD) proteins and a suitable pharmaceutical carrier, comprising the following steps:

   a. isolating enamel matrix proteins and/or enamel matrix derivative (EMD) proteins from a developing mammals teeth,
   b. solving said isolate in a suitable pharmaceutical carrier,
   c. lowering the pH of said formulation initially to below pH 4, and
   d. heating said formulation to between 40-100°C for at least 10 minutes.

2. A process for producing a pharmaceutical, dental and/or cosmetic formulation comprising enamel matrix proteins and/or enamel matrix derivative (EMD) proteins and a suitable pharmaceutical carrier according to claim 1, wherein the pH of said formulation is in step c. lowered to below 3.5, such as to at most 3.5, such as to 2.

3. A process for producing a pharmaceutical, dental and/or cosmetic formulation comprising enamel matrix proteins and/or enamel matrix derivative (EMD) proteins and a suitable pharmaceutical carrier according to claim 1 or 2, wherein the formulation of step c. is in step d. heated to at least 50 °C for at least 1 hour, such as heated to at least 80°C for at least 3 hours.

4. A process for producing a pharmaceutical, dental and/or cosmetic formulation comprising enamel matrix proteins and/or enamel matrix derivative (EMD) proteins and a suitable pharmaceutical carrier according to claim 1 or 2, wherein the suitable pharmaceutical carrier is selected from the group consisting of a universal buffer, aqueous acetic acid, citric acid, and $H_2O$.

5. A process for producing a pharmaceutical, dental and/or cosmetic formulation comprising enamel matrix proteins and/or enamel matrix derivative (EMD) proteins and a suitable pharmaceutical carrier according to any one of claims1-4, wherein the enamel matrix proteins and/or enamel matrix derivative (EMD) proteins comprise at least 60-70% amelogenin, having an average molecular weight selected from the group consisting of between 18 and 25 kDa, between 20 and 24 kDa, between 20 and 22 kDa, and 20 kDa.

6. A process for producing a pharmaceutical, dental and/or cosmetic formulation comprising enamel matrix proteins and/or enamel matrix derivative (EMD) proteins and a suitable pharmaceutical carrier according to any one of claims1-5, which further comprises mixing the formulation of step d. with a suitable viscosity modifier.

7. A process for producing a pharmaceutical, dental and/or cosmetic formulation comprising enamel matrix proteins and/or enamel matrix derivative (EMD) proteins and a suitable pharmaceutical carrier according to claim 6, which further comprises adjusting the pH of the formulation of step d. to at least pH5 before mixing said formulation with a suitable viscosity modifier.

8. A process for producing a pharmaceutical, dental and/or cosmetic formulation comprising enamel matrix proteins and/or enamel matrix derivative (EMD) proteins and a suitable pharmaceutical carrier according to any one of claims 6 or 7,wherein the viscosity modifier is PGA.

9. A process for producing a pharmaceutical, dental and/or cosmetic formulation comprising enamel matrix proteins and/or enamel matrix derivative (EMD) proteins and a suitable pharmaceutical carrier according to claim 7, wherein the PGA is e-beam sterilized PGA and wherein the formulation has a pH of at least pH5.

10. A process for producing a pharmaceutical, dental and/or cosmetic formulation comprising enamel matrix proteins and/or enamel matrix derivative (EMD) proteins and a suitable pharmaceutical carrier according to claim 7 or 8, wherein the PGA is sterilized propylene glycol alginate (PGA) with a weight average molecular weight above 130 kDa.

11. A process for producing a pharmaceutical, dental and/or cosmetic formulation comprising enamel matrix proteins and/or enamel matrix derivative (EMD) proteins and a suitable pharmaceutical carrier according to claims 1-9, which further comprises adjusting the pH of the formulation of step d. to at the most pH 5, such as to between pH 2 and pH 5, such as between pH 2.5 and pH 4.5, or such as to pH 2.5.

12. A process for producing a pharmaceutical, dental and/or cosmetic formulation comprising enamel matrix proteins and/or enamel matrix derivative (EMD) proteins and a suitable pharmaceutical carrier according to any one of claim 11, wherein said formulation is mixed with a suitable viscosity modifier.

13. A process for producing a pharmaceutical, dental and/or cosmetic formulation comprising enamel matrix proteins and/or enamel matrix derivative (EMD) proteins and a suitable pharmaceutical carrier according to any one of claims 11 or 12, wherein the viscosity modifier is hyaluronic acid, such as e-beam sterilized Hyaluronic acid.

14. A pharmaceutical, dental and/or cosmetic formulation comprising enamel matrix proteins and/or enamel matrix derivative (EMD) proteins and a suitable pharmaceutical carrier produced by a process according to any one of claims 1-13, which is **characterized by** a shelf life and/or durability of at least 12 months at 2°C-RT.

15. A pharmaceutical, dental and/or cosmetic formulation comprising enamel matrix proteins and/or enamel matrix derivative (EMD) proteins and a suitable pharmaceutical carrier according to claim 14, further comprising arginine.

16. A pharmaceutical, dental and/or cosmetic formulation according to claim 15, wherein said arginine has a concentration of at the most 500 mM.

17. A pharmaceutical, dental and/or cosmetic formulation according to any one of claims 13-16, further comprising a suitable bone ceramic and/or a bonegraft.

18. A pharmaceutical, dental and/or cosmetic formulation according to any one of claims 13-17, for use in medicine.

19. A pharmaceutical, dental and/or cosmetic formulation according to any one of claims 13-17, for use in wound-treatment and/or in treatment of periimplant diseases and/or periodontitis.

20. A kit comprising a pharmaceutical, dental and/or cosmetic formulation according to any one of claims 13-17, and at least one further component selected from the group consisting of granules, bone ceramics, scaffolds, a bonegraft, natural bone material, a bone block, artificial teeth, and an implant.

**Patentansprüche**

1. Verfahren zum Herstellen einer pharmazeutischen, dentalen und/oder kosmetischen Formulierung, die Zahnschmelzmatrixproteine und/oder Zahnschmelzmatrixderivat (EMD)-Proteine und einen geeigneten pharmazeutischen Träger umfasst, umfassend die folgenden Schritte:

    a. Isolieren von Zahnschmelzmatrixproteinen und/oder Zahnschmelzmatrixderivat (EMD)-Proteinen von einem sich entwickelnden Säugetierzahn,
    b. Lösen des Isolats in einem geeigneten pharmazeutischen Träger,
    c. Herabsetzen des pH-Werts der Formulierung anfänglich auf unter pH 4, und
    d. Erwärmen der Formulierung auf zwischen 40-100°C für zumindest 10 Minuten.

2. Verfahren zum Herstellen einer pharmazeutischen, dentalen und/oder kosmetischen Formulierung, die Zahnschmelzmatrixproteine und/oder Zahnschmelzmatrixderivat (EMD)-Proteine und einen geeigneten pharmazeutischen Träger umfasst, nach Anspruch 1, wobei der pH-Wert der Formulierung in Schritt c. auf unter 3,5 herabgesetzt wird, wie beispielsweise auf höchstens 3,5, wie beispielsweise auf 2.

3. Verfahren zum Herstellen einer pharmazeutischen, dentalen und/oder kosmetischen Formulierung, die Zahnschmelzmatrixproteine und/oder Zahnschmelzmatrixderivat (EMD)-Proteine und einen geeigneten pharmazeutischen Träger umfasst, nach Anspruch 1 oder 2, wobei die Formulierung von Schritt c. in Schritt d. auf zumindest 50°C für zumindest 1 Stunde erwärmt wird, wie beispielsweise auf zumindest 80°C für zumindest 3 Stunden erwärmt wird.

4. Verfahren zum Herstellen einer pharmazeutischen, dentalen und/oder kosmetischen Formulierung, die Zahnschmelzmatrixproteine und/oder Zahnschmelzmatrixderivat (EMD)-Proteine und einen geeigneten pharmazeutischen Träger umfasst, nach Anspruch 1 oder 2, wobei der geeignete pharmazeutische Träger ausgewählt ist, aus der Gruppe, bestehend aus einem universellen Puffer, wässrige Essigsäure, Zitronensäure und $H_2O$.

5. Verfahren zum Herstellen einer pharmazeutischen, dentalen und/oder kosmetischen Formulierung, die Zahnschmelzmatrixproteine und/oder Zahnschmelzmatrixderivat (EMD)-Proteine und einen geeigneten pharmazeutischen Träger umfasst, nach einem der Ansprüche 1-4, wobei die Zahnschmelzmatrixproteine und/oder die Zahnschmelzmatrixderivat (EMD)-Proteine zumindest 60-70% Amelogenin umfassen, das ein durchschnittliches Molekulargewicht, ausgewählt aus der Gruppe, bestehend aus zwischen 18 und 25 kDa, zwischen 20 und 24 kDa, zwischen 20 und 22 kDa und 20 kDa, aufweist.

6. Verfahren zum Herstellen einer pharmazeutischen, dentalen und/oder kosmetischen Formulierung, die Zahnschmelzmatrixproteine und/oder Zahnschmelzmatrixderivat (EMD)-Proteine und einen geeigneten pharmazeutischen Träger umfasst, nach einem der Ansprüche 1-5, das weiter ein Mischen der Formulierung von Schritt d. mit einem geeigneten Viskositätsmodifikator umfasst.

**7.** Verfahren zum Herstellen einer pharmazeutischen, dentalen und/oder kosmetischen Formulierung, die Zahnschmelzmatrixproteine und/oder Zahnschmelzmatrixderivat (EMD)-Proteine und einen geeigneten pharmazeutischen Träger umfasst, nach Anspruch 6, das weiter ein Einstellen des pH-Werts der Formulierung von Schritt d. auf zumindest pH 5 vor dem Mischen der Formulierung mit einem geeigneten Viskositätsmodifikator umfasst.

**8.** Verfahren zum Herstellen einer pharmazeutischen, dentalen und/oder kosmetischen Formulierung, die Zahnschmelzmatrixproteine und/oder Zahnschmelzmatrixderivat (EMD)-Proteine und einen geeigneten pharmazeutischen Träger umfasst, nach Anspruch 6 oder 7, wobei der Viskositätsmodifikator PGA ist.

**9.** Verfahren zum Herstellen einer pharmazeutischen, dentalen und/oder kosmetischen Formulierung, die Zahnschmelzmatrixproteine und/oder Zahnschmelzmatrixderivat (EMD)-Proteine und einen geeigneten pharmazeutischen Träger umfasst, nach Anspruch 7, wobei das PGA ein Elektronenstrahl-sterilisiertes PGA ist und wobei die Formulierung einen PH-Wert von zumindest pH 5 aufweist.

**10.** Verfahren zum Herstellen einer pharmazeutischen, dentalen und/oder kosmetischen Formulierung, die Zahnschmelzmatrixproteine und/oder Zahnschmelzmatrixderivat (EMD)-Proteine und einen geeigneten pharmazeutischen Träger umfasst, nach Anspruch 7 oder 8, wobei das PGA ein sterilisiertes Propylenglycolalginat (PGA) mit einem gewichtsmäßigem durchschnittlichen Molekulargewicht über 130 kDa ist.

**11.** Verfahren zum Herstellen einer pharmazeutischen, dentalen und/oder kosmetischen Formulierung, die Zahnschmelzmatrixproteine und/oder Zahnschmelzmatrixderivat (EMD)-Proteine und einen geeigneten pharmazeutischen Träger umfasst, nach einem der Ansprüche 1-9, das weiter ein Einstellen des pH-Werts der Formulierung von Schritt d. auf höchstens pH 5, wie beispielsweise auf zwischen pH 2 und pH 5, wie beispielsweise zwischen pH 2,5 und pH 4,5 oder wie beispielsweise auf pH 2,5 umfasst.

**12.** Verfahren zum Herstellen einer pharmazeutischen, dentalen und/oder kosmetischen Formulierung, die Zahnschmelzmatrixproteine und/oder Zahnschmelzmatrixderivat (EMD)-Proteine und einen geeigneten pharmazeutischen Träger umfasst, nach einem der Ansprüche 11, wobei die Formulierung mit einem geeigneten Viskositätsmodifikator gemischt wird.

**13.** Verfahren zum Herstellen einer pharmazeutischen, dentalen und/oder kosmetischen Formulierung, die Zahnschmelzmatrixproteine und/oder Zahnschmelzmatrixderivat (EMD)-Proteine und einen geeigneten pharmazeutischen Träger umfasst, nach einem der Ansprüche 11 oder 12, wobei der geeignete Viskositätsmodifikator Hyaluronsäure ist, wie beispielsweise Elektronenstrahl-sterilisierte Hyaluronsäure.

**14.** Pharmazeutische, dentale und/oder kosmetische Formulierung, die Zahnschmelzmatrixproteine und/oder Zahnschmelzmatrixderivat (EMD)-Proteine und einen geeigneten pharmazeutischen Träger umfasst, die durch ein Verfahren nach einem der Ansprüche 1-13 hergestellt wird und die durch eine Haltbarkeit und/oder Beständigkeit von zumindest 12 Monaten bei 2°C-RT gekennzeichnet ist.

**15.** Pharmazeutische, dentale und/oder kosmetische Formulierung, die Zahnschmelzmatrixproteine und/oder Zahnschmelzmatrixderivat (EMD)-Proteine und einen geeigneten pharmazeutischen Träger umfasst, nach Anspruch 14, weiter umfassend Arginin.

**16.** Pharmazeutische, dentale und/oder kosmetische Formulierung nach Anspruch 15, wobei das Arginin eine Konzentration von höchstens 500 mM aufweist.

**17.** Pharmazeutische, dentale und/oder kosmetische Formulierung nach einem der Ansprüche 13-16, weiter umfassend eine geeignete Knochenkeramik und/oder ein Knochentransplantat.

**18.** Pharmazeutische, dentale und/oder kosmetische Formulierung nach einem der Ansprüche 13-17 zur Verwendung in der Medizin.

**19.** Pharmazeutische, dentale und/oder kosmetische Formulierung nach einem der Ansprüche 13-17 zur Verwendung bei der Wundbehandlung und/oder bei der Behandlung von periimplantären Erkrankungen und/oder Parodontitis.

**20.** Kit, umfassend eine pharmazeutischen, dentalen und/oder kosmetischen Formulierung nach einem der Ansprüche 13-17 und zumindest eine weitere Komponente, ausgewählt aus der Gruppe, bestehend aus Granulaten, Knochenkeramiken, Gerüsten, einem Knochentransplantat, natürlichem Knochenmaterial, einem Knochenblock, künstlichen Zähnen und einem Implantat.

**Revendications**

1. Procédé pour produire une formulation pharmaceutique, dentaire et/ou cosmétique comprenant des protéines de matrice de l'émail et/ou des protéines de dérivé de matrice de l'émail (EMD) et un support pharmaceutique approprié, comprenant les étapes suivantes :

   a. l'isolement de protéines de matrice de l'émail et/ou de protéines de dérivé de matrice de l'émail (EMD) à partir de dents de mammifère en développement,
   b. la mise en solution dudit isolat dans un support pharmaceutique approprié,
   c. la diminution du pH de ladite formulation initialement en dessous de pH 4, et
   d. le chauffage de ladite formulation entre 40 et 100 °C pendant au moins 10 minutes.

2. Procédé pour produire une formulation pharmaceutique, dentaire et/ou cosmétique comprenant des protéines de matrice de l'émail et/ou des protéines de dérivé de matrice de l'émail (EMD) et un support pharmaceutique approprié selon la revendication 1, dans lequel le pH de ladite formulation est dans l'étape c. diminué en dessous de 3,5, tel qu'au plus à 3,5, tel qu'à 2.

3. Procédé pour produire une formulation pharmaceutique, dentaire et/ou cosmétique comprenant des protéines de matrice de l'émail et/ou des protéines de dérivé de matrice de l'émail (EMD) et un support pharmaceutique approprié selon la revendication 1 ou 2, dans lequel la formulation de l'étape c. est dans l'étape d. chauffée à au moins 50 °C pendant 1 heure, telle que chauffée à au moins 80 °C pendant au moins 3 heures.

4. Procédé pour produire une formulation pharmaceutique, dentaire et/ou cosmétique comprenant des protéines de matrice de l'émail et/ou des protéines de dérivé de matrice de l'émail (EMD) et un support pharmaceutique approprié selon la revendication 1 ou 2, dans lequel le support pharmaceutique approprié est sélectionné dans le groupe constitué d'un tampon universel, de l'acide acétique aqueux, de l'acide citrique, et de $H_2O$.

5. Procédé pour produire une formulation pharmaceutique, dentaire et/ou cosmétique comprenant des protéines de matrice de l'émail et/ou des protéines de dérivé de matrice de l'émail (EMD) et un support pharmaceutique approprié selon l'une quelconque des revendications 1 à 4, dans lequel les protéines de matrice de l'émail et/ou les protéines de dérivé de matrice de l'émail (EMD) comprennent au moins 60 à 70% d'amélogénine, ayant un poids moléculaire moyen sélectionné dans le groupe constitué de entre 18 et 25 kDa, entre 20 et 24 kDa, entre 20 et 22 kDa, et 20 kDa.

6. Procédé pour produire une formulation pharmaceutique, dentaire et/ou cosmétique comprenant des protéines de matrice de l'émail et/ou des protéines de dérivé de matrice de l'émail (EMD) et un support pharmaceutique approprié selon l'une quelconque des revendications 1 à 5, qui comprend en outre le mélange de la formulation de l'étape d. avec un modificateur de viscosité approprié.

7. Procédé pour produire une formulation pharmaceutique, dentaire et/ou cosmétique comprenant des protéines de matrice de l'émail et/ou des protéines de dérivé de matrice de l'émail (EMD) et un support pharmaceutique approprié selon la revendication 6, qui comprend en outre l'ajustement du pH de la formulation de l'étape d. à au moins pH 5 avant le mélange de ladite formulation avec un modificateur de viscosité approprié.

8. Procédé pour produire une formulation pharmaceutique, dentaire et/ou cosmétique comprenant des protéines de matrice de l'émail et/ou des protéines de dérivé de matrice de l'émail (EMD) et un support pharmaceutique approprié selon l'une quelconque des revendications 6 ou 7, dans lequel le modificateur de viscosité est un PGA.

9. Procédé pour produire une formulation pharmaceutique, dentaire et/ou cosmétique comprenant des protéines de matrice de l'émail et/ou des protéines de dérivé de matrice de l'émail (EMD) et un support pharmaceutique approprié selon la revendication 7, dans lequel le PGA est un PGA stérilisé par un faisceau d'électrons et dans lequel la formulation présente un pH d'au moins pH 5.

10. Procédé pour produire une formulation pharmaceutique, dentaire et/ou cosmétique comprenant des protéines de matrice de l'émail et/ou des protéines de dérivé de matrice de l'émail (EMD) et un support pharmaceutique approprié selon la revendication 7 ou 8, dans lequel le PGA est un alginate de propylène glycol (PGA) stérilisé ayant un poids moléculaire moyen en poids au-dessus de 130 kDa.

11. Procédé pour produire une formulation pharmaceutique, dentaire et/ou cosmétique comprenant des protéines de matrice de l'émail et/ou des protéines de dérivé de matrice de l'émail (EMD) et un support pharmaceutique approprié selon les revendications 1 à 9, qui comprend en outre l'ajustement du pH de la formulation de l'étape d. à au plus pH 5, tel qu'à entre pH 2 et pH 5, tel qu'à entre pH 2,5 et pH 4,5, ou tel qu'à pH 2,5.

12. Procédé pour produire une formulation pharmaceutique, dentaire et/ou cosmétique comprenant des protéines de matrice de l'émail et/ou des protéines de dérivé de matrice de l'émail (EMD) et un support pharmaceutique approprié selon l'une quelconque de la revendication 11, dans lequel ladite formulation est mélangée avec un modificateur de viscosité approprié.

13. Procédé pour produire une formulation pharmaceutique, dentaire et/ou cosmétique comprenant des protéines de matrice de l'émail et/ou des protéines de dérivé de matrice de l'émail (EMD) et un support pharmaceutique approprié selon l'une quelconque des revendications 11 ou 12, dans lequel le modificateur de viscosité est un acide hyaluronique, tel qu'un acide hyaluronique stérilisé par un faisceau d'électrons.

14. Formulation pharmaceutique, dentaire et/ou cosmétique comprenant des protéines de matrice de l'émail et/ou des protéines de dérivé de matrice de l'émail (EMD) et un support pharmaceutique approprié produite par un procédé selon l'une quelconque des revendications 1 à 13, qui est **caractérisée par** une durée de conservation et/ou une durabilité d'au moins 12 mois à 2 °C-TA.

15. Formulation pharmaceutique, dentaire et/ou cosmétique comprenant des protéines de matrice de l'émail et/ou des protéines de dérivé de matrice de l'émail (EMD) et un support pharmaceutique approprié selon la revendication 14, comprenant en outre de l'arginine.

16. Formulation pharmaceutique, dentaire et/ou cosmétique selon la revendication 15, dans laquelle ladite arginine présente une concentration d'au plus 500 mM.

17. Formulation pharmaceutique, dentaire et/ou cosmétique selon l'une quelconque des revendications 13 à 16, comprenant en outre une céramique osseuse appropriée et/ou un greffon osseux.

18. Formulation pharmaceutique, dentaire et/ou cosmétique selon l'une quelconque des revendications 13 à 17, pour une utilisation en médecine.

19. Formulation pharmaceutique, dentaire et/ou cosmétique selon l'une quelconque des revendications 13 à 17, pour une utilisation dans un traitement d'une blessure et/ou dans un traitement de maladies péri-implantaires et/ou d'une parodontite.

20. Kit comprenant une formulation pharmaceutique, dentaire et/ou cosmétique selon l'une quelconque des revendications 13 à 17, et au moins un composant supplémentaire sélectionné dans le groupe constitué des granulés, des céramiques osseuses, des supports, d'un greffon osseux, d'un matériau osseux naturel, d'un bloc osseux, des dents artificielles, et d'un implant.

Fig.1 A/B

Fig1 C/D

Fig.2 A/B

Fig.2C/D

Fig.2E/F

Fig.3

Fig4 A/B

Fig4 C/D

Fig4 E/F

Fig 5

Fig 6 A/B

Fig 6 C/D

Fig 6 E/F

Fig 7

Fig. 8

| | EMD (0.5 mg/mL) | | | | EMD/PGA (0.5/1 mg/mL) | | | |
|---|---|---|---|---|---|---|---|---|
| | EMD+ Arg pH 5 | EMD+ Arg pH 2 | EMD pH 5 | EMD pH 2 | EMD+ Arg pH 5 | EMD+ Arg pH 2 | EMD pH 5 | EMD pH 2 |
| $A_0$ | 46 | 48 | 35.9 | 48.7 | 56.5 | 59.4 | 46.1 | 55.2 |
| $A_1$ | 10.3 | 15.2 | 15.2 | 16.83 | 9.00 | 10.7 | 11.7 | 12.6 |
| $\tau_1$ | 0.32 | 0.37 | 0.32 | 0.28 | 0.40 | 0.34 | 0.33 | 0.26 |
| $A_2$ | 21.8 | 20.1 | 22.3 | 18.3 | 14.4 | 16.6 | 18.3 | 17.1 |
| $\tau_2$ | 3.24 | 3.32 | 3.22 | 2.85 | 3.72 | 3.47 | 3.46 | 3.14 |

Fig. 9

Fig. 10

Fig. 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4672032 A, Slavkin
- EP 0337967 B
- EP 0263086 B
- EP 1059934 A
- EP 01201915 A
- WO 0664381 A
- WO 0197834 A
- WO 0053197 A
- WO 0053196 A
- WO 03024479 A
- WO 02080994 A
- US 4672032 B

### Non-patent literature cited in the description

- **GESTRELIUS S ; LYNGSTADAAS SP.** Hammarstrøm L. Emdogain - periodontal regeneration based on biomimicry. *Clin Oral Invest,* 2000, vol. 4, 120-125
- **HAMMARSTRÖM et al.** *Journal of Clinical Periodontology,* 1997, vol. 24, 658-668
- **LYNGSTADAAS et al.** *Journal of Clinical Periodontology,* 2001, vol. 28, 181-188
- **CASAL HL ; KOHLER U ; MANTSCH HH.** Structural and conformational changes of β-lactoglobulin B : an infrared spectroscopic study of the effect of pH and temperature. *Biochimica et Biohysica Acta (BBA)-Protein structure and Molecular Enzymology,* 1998, vol. 957 (1), 11-20
- **SAMBROOK, J. et al.** Molecular Cloning. Cold Spring Harbor Laboratory Press, 1989
- **TEN CATE.** *Oral Histology,* 1994
- **ROBINSON.** *Eur. J. Oral Science,* January 1998, vol. 106 (1), 282-91
- **DAYHOFF ; SCHWARTZ ; ORCUTT.** *Atlas Protein Seq. Struc.,* 1978, vol. 5, 345-352
- **HENIKOFF AND HENIKOFF.** *Proc Natl Acad Sci U S A,* 1992, vol. 89 (22), 10915-9
- Remington's Pharmaceutical Sciences'' and ''Encyclopedia of Pharmaceutical Technology. Marcel Dekker, Inc, 1988
- **BLOCK, I. D. ; SCHEFFOLD, F.** Modulated 3D cross-correlation light scattering: Improving turbid sample characterization. *Review of Scientific Instruments,* 2010, vol. 81, 123107-7
- **BARTH, I. GRILLO ; M. GRADZIELSKI.** Dynamics of formation of vesicles studied by highly time-resolved stopped-flow experiments. *Tenside Surf. Det.,* 2010, vol. 47, 300-306
- **LAMBRIGHT DG ; BALASUBRAMANIAN S ; BOXER SG.** Protein relaxation dynamics in human myoglobin. *Chemical Physics,* 1991, vol. 158, 249-260
- **BARTH A ; ZSCHERP C.** What vibrations tell us about proteins. *Quarterly Reviews of Biophysics,* 2002, vol. 35 (4), 369-430
- **FANGA PA ; CONWAYB JF ; MARGOLISC HC ; SIMMERD JP ; BENIASHA E.** Hierarchical self- assembly of amelogenin and the regulation of biomineralization at the nanoscale. *Pnas,* 2011, vol. 108 (34), 14097-14102
- **SAKAMOTO R ; NISHIKORI S ; SHIRAKI K.** High temperature increases the refolding yield of reduced lysozyme: implication for the productive process for folding. *Biotechonl. Prog,* 2004, vol. 20, 1128-1133.15
- **PETROU I ; HEU R ; STRANICK M ; LAVANDER S ; ZAIDEL L ; CUMMINS D et al.** A breakthrough therapy for dentin hypersensitivity: how dental products containing 8% arginine and calcium carbonate work to deliver effective relief of sensisive teeth. *The journal of Clinical Dentistry,* 2009, vol. 20 (1), 23-31
- **GESTRELIUS S ; LYNGSTADAAS SP ; HAMMARSTRØM L.** Emdogain - periodontal regeneration based on biomimicry. *Clin Oral Invest,* 2000, vol. 4, 120-125
- **HENIKOFF AND HENIKOFF.** *Proc Natl Acad Sci USA,* 1992, vol. 89 (22), 10915-9
- REMINGTON'S PHARMACEUTICAL SCIENCES ENCYCLOPEDIA OF PHARMACEUTICAL TECHNOLOGY. Marcel Dekker, Inc, 1988
- **KONG J ; YU S.** Fourier Transform Infrared Spectroscopic Analysis of Protein Secondary Structures. *Acta Biochimica et Biophysica Sinica,* 2007, vol. 39 (8), 549-559

- **HERBERHOLD H ; MARCHAL S ; LANGE R ; SCHEYHING CH ; VOGELAND RF ; WINTER R.** Characterization of the Pressure-induced Intermediate and Unfolded State of Red-shifted Green Fluorescent Protein-A Static and Kinetic FTIR, UV/VIS and Fluorescence Spectroscopy Study. *J. Mol. Biol.,* 2003, vol. 330, 1153-1164

- **BARTH ; I. GRILLO ; M. GRADZIELSKI.** Dynamics of formation of vesicles studied by highly time-resolved stopped-flow experiments. *Tenside Surf. Det.,* 2010, vol. 47, 300-306
- **SCHNEIDER, CP ; SHUKLA, D ; TROUT BL.** Arginine and Hofmeister series, the role of ion-ion interactions in protein aggregation suppressoipn. *J. Phys.Chem.B,* 2011, vol. 115, 7447-7458